(19) Europäisches Patentamt
European Patent Office
Office européen des brevets

(11) Veröffentlichungsnummer: **0 171 544**
**B1**

(12) # EUROPÄISCHE PATENTSCHRIFT

(45) Veröffentlichungstag der Patentschrift: **04.01.89**

(21) Anmeldenummer: **85107541.6**

(22) Anmeldetag: **19.06.85**

(51) Int. Cl.⁴: **C 07 D 231/40**, A 01 N 47/18, A 01 N 47/36 // C07D231/38, C07C109/04, C07C121/82

(54) **Substituierte 5-Acylamino-1-phenylpyrazole.**

(30) Priorität: **27.06.84 DE 3423582**

(43) Veröffentlichungstag der Anmeldung: **19.02.86 Patentblatt 86/8**

(45) Bekanntmachung des Hinweises auf die Patenterteilung: **04.01.89 Patentblatt 89/1**

(84) Benannte Vertragsstaaten: **AT BE CH DE FR GB IT LI NL**

(56) Entgegenhaltungen:
**EP-A-0 034 945**
**EP-A-0 053 698**
**EP-A-0 071 794**
**WO-A-83/00331**
**GB-A-2 101 999**
**US-A-3 646 059**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Gehring, Reinhold, Dr., Dasnöckel 49, D-5600 Wuppertal 11 (DE)**
Erfinder: **Stetter, Jörg, Dr., Gellertweg 4, D-5600 Wuppertal 1 (DE)**
Erfinder: **Schallner, Otto, Dr., Noldeweg 22, D-4019 Monheim (DE)**
Erfinder: **Eue, Ludwig, Dr., Paul- Klee- Strasse 36, D-5090 Leverkusen 1 (DE)**
Erfinder: **Santel, Hans- Joachim, Dr., Gerstenkamp 19, D-5000 Köln 80 (DE)**
Erfinder: **Schmidt, Robert R. Dr., Im Waldwinkel 110, D-5060 Bergisch- Gladbach (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft neue substituierte 5-Acylamino-1-phenylpyrazole, mehrere Verfahren zu ihrer Herstellung und ihre Verwendung als Herbizide.

Es ist bereits bekannt, daß bestimmte substituierte 5-Acylamino-1-phenylpyrazole, wie beispielsweise das 4-Cyano-5-propionamido-1-(2,4,6-trichlorphenyl)-pyrazol, herbizide Eigenschaften besitzen (vgl. z. B. DE-OS-3 226 513).

Aus der GB-A-2 101 999 sind 5-Acylamino-1-phenylpyrazole bekannt, welche sich jedoch durch das Substitutionsmuster am Phenylring von den beanspruchten Verbindungen unterscheiden.

Die herbizide Wirkung dieser bekannten Verbindungen gegenüber Unkräutern, ebenso wie deren Verträglichkeit gegenüber wichtigen Kulturpflanzen ist jedoch nicht immer in allen Anwendungsgebieten völlig zufriedenstellend.

Es wurden neue substituierte 5-Acylamino-1-phenylpyrazole der allgemeinen Formel (I) gefunden,

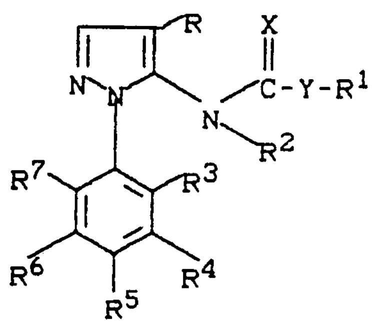

(I)

in welcher

R für Cyano steht,

$R^1$ für jeweils geradkettiges oder verzweigtes Alkyl, Alkenyl, Alkinyl, Alkoxyalkyl oder Alkylthioalkyl, mit jeweils bis zu 6 Kohlenstoffatomen in den einzelnen Alkyl- bzw. Alkenyl- oder Alkinylteilen steht, außerdem für geradkettges oder verzweigtes Halogenalkyl mit 1 bis 6 Kohlenstoffatomen und 1 bis 9 gleichen oder verschiedenen Halogenatomen steht, ferner für cycloalkyl mit 3 bis 7 Kohlenstoffatomen oder für gegebenenfalls einfach oder mehrfach, gleich oder verschieden substituiertes Aryl mit 6 bis 10 Kohlenstoffatomen steht, wobei als Substituenten in Frage kommen; Halogen, Cyano, Nitro, jeweils geradkettiges oder verzweigtes Alkyl, Alkoxy, Alkylthio oder Alkoxycarbonyl mit jeweils bis zu 4 Kohlenstoffatomen in den einzelnen Alkylteilen,

oder für den Fall, daß Y für die

-N-Gruppe steht,
|
Z

auch für Wasserstoff steht,

X für Sauerstoff oder Schwefel steht,

Y für Sauerstoff, Schwefel oder die

-N-Gruppe steht,
|
Z

wobei Z für Wasserstoff, für jeweils geradkettiges oder verzweigtes Alkyl, Alkoxy, Alkenyl oder Alkinyl mit jeweils bis zu 4 Kohlenstoffatomen oder für gegebenenfalls einfach oder mehrfach gleich oder verschieden durch Halogen, Cyano, Nitro, jeweils geradkettiges oder verzweigtes Alkyl, Alkoxy, Alkylthio oder Alkoxycarbonyl mit jeweils bis zu 4 Kohlenstoffatomen in den einzelnen Alkylteilen substituiertes Aryl mit 6 bis 10 Kohlenstoffatomen steht,

$R^2$ für Wasserstoff oder den Rest

X
||
-C-Y-$R^1$ steht und

$R^3$, $R^4$, $R^5$, $R^6$ und $R^7$ unabhängig voneinander für Wasserstoff, Fluor, Chlor, Brom, Iod, Nitro, jeweils geradkettiges oder verzweigtes Alkyl, Alkoxy, Alkylsulfonyl oder Alkoxycarbonyl mit bis zu 4 Kohlenstoffatomen in den jeweiligen Alkylteilen oder für einen Rest- $(X')_n$-$R^8$ stehen, wobei

X' für Sauerstoff, Schwefel, Sulfinyl oder Sulfonyl steht,

n für 0 oder 1 steht und $R^8$ für geradkettiges oder verzweigtes Halogenalkyl mit bis zu 4 Kohlenstoffatomen und bis zu 9 gleichen oder verschiedenen Halogenatomen steht, mit der Maßgabe, daß mindestens einer der Reste $R^3$, $R^4$, $R^5$, $R^6$ oder $R^7$ für einen Rest $-(X')_n-R^8$ steht, wobei jedoch nicht gleichzeitig R für cyano und $R^5$ für Trifluormethyl stehen.

Weiterhin wurde gefunden, daß man die neuen substituierten 5-Acylamino-1-phenylpyrazole der allgemeinen Formel (I) erhält, wenn man

(a) 5-Amino-pyrazole der Formel (II)

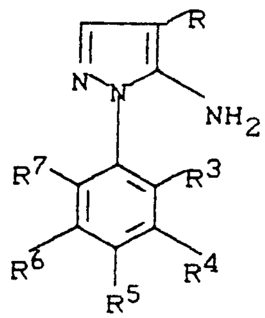

(II)

in welcher

R, $R^3$, $R^4$, $R^5$, $R^6$ und $R^7$ die oben angegebene Bedeutung haben,

(α) mit Acylierungsmittel der Formel (III)

$$R^1-Y-\underset{\underset{X}{\parallel}}{C}-A \qquad (III)$$

in welcher

$R^1$, X und Y die oben angegebene Bedeutung haben und

A für Halogen steht,

gegebenenfalls in Gegenwart eines Verdünnungsmittels und gegebenenfalls in Gegenwart eines Säurebindemittels umsetzt, oder

(β) mit Iso(thio)cyanaten der Formel (IV)

$$R^1-N=C=X \qquad (IV)$$

in welcher

$R^1$ und X die oben angegebene Bedeutung haben,

ebenfalls gegebenenfalls in Gegenwart eines Verdünnungsmittels und gegebenenfalls in Gegenwart eines basischen Katalysators umsetzt, oder wenn man

(b) die nach Verfahren (a - α) erhältlichen Biscarbamate der Formel (Ia)

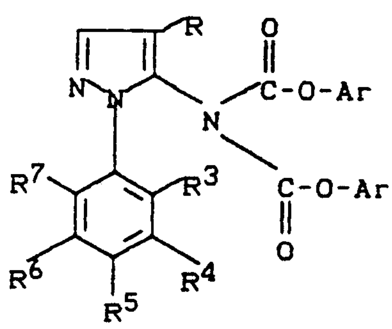

(Ia)

in welcher

Ar für gegebenenfalls ein- bis dreifach gleich oder verschieden durch Halogen, Cyano, Nitro, Methyl, Ethyl, Methoxy, Ethoxy oder Trifluormethyl substituiertes Phenyl steht und

R, $R^3$, $R^4$, $R^5$, $R^6$ und $R^7$ die oben angegebene Bedeutung haben,

mit Alkoholen, Aminen oder Thiolen der Formel (V)

$$R^1-Y-H \qquad (V)$$

in welcher

$R^1$ und Y die oben angegebene Bedeutung haben,

gegebenenfalls in Gegenwart eines Verdünnungsmittels und gegebenenfalls in Gegenwart eines basischen Katalysators umsetzt.

Schließlich wurde gefunden, daß die neuen substituierten 5-Acylamino-1-phenylpyrazole der Formel (I) herbizide Eigenschaften, insbesondere auch selektiv-herbizide Eigenschaften besitzen.

Überraschenderweise besitzen die neuen substituierten 5-Acylamino-1-phenyl-pyrazole der Formel (I) eine bessere herbizide Wirksamkeit gegenüber Schadpflanzen, bei gleichzeitiger besserer Verträglichkeit gegenüber wichtigen Nutzpflanzen, als beispielsweise das aus dem Stand der Technik bekannte 4-Cyano-5-propionamido-1-(2,4,6-trichlorphenyl)-pyrazol, welches eine chemisch und wirkungsmäßig naheliegende Verbindung ist.

Die neuen substituierten 5-Acyl-amino-1-phenylpyrazole sind durch die Formel (I) allgemein definiert. Bevorzugt sind Verbindungen der Formel (I), bei welchen

R für Cyano steht,

$R^1$ für Methyl, Ethyl, n- und i-Propyl, n-, i-, s- und t-Butyl, Cyclopropyl, 2,2-Dichlorcyclopropyl, 2,2-Dichlor-1-methylcyclopropyl, Methylthiomethyl, Cyclopentyl, Cyclohexyl, Ethoxymethyl, Methoxymethyl, Ethoxyethyl, Chlormethyl, Dichlormethyl, Trichlormethyl, Trifluormethyl, Dichlorfluormethyl, Difluorchlormethyl oder Pentafluorethyl, oder für gegebenenfalls ein- bis dreifach, gleich oder verschieden durch Fluor, Chlor, Brom, Nitro, Methyl oder Methoxy substituiertes Phenyl steht, oder für den Fall, daß Y für die

-N-Gruppe steht, auch für Wasserstoff steht,
 |
 Z

X für Sauerstoff oder Schwefel steht,

Y für Sauerstoff, Schwefel oder die

-N-Gruppe steht,
 |
 Z

wobei Z für Wasserstoff, Methyl, Ethyl, n- oder i-Propyl, n-, i-, s- oder t-Butyl, Methoxy, Ethoxy, Allyl, Propargyl oder für gegebenenfalls ein- bis dreifach gleich oder verschieden substituiertes Phenyl steht, wobei als Phenylsubstituenten die bei $R^1$ genannten in Frage kommen,

$R^2$ für Wasserstoff oder den Rest

$$-\overset{X}{\overset{\|}{C}}-Y-R^1$$ steht und

$R^3$, $R^4$, $R^5$, $R^6$ und $R^7$ unabhängig voneinander für Wasserstoff, Fluor, Chlor, Brom, Cyano, Nitro, Methyl, Ethyl, n- und i-Propyl, Methoxy, Ethoxy, Methylsulfonyl, Methoxycarbonyl, Ethoxycarbonyl oder für einen Rest $-(X')_n-R^8$ stehen, wobei X' für Sauerstoff, Schwefel, Sulfinyl oder Sulfonyl steht, n für 0 oder 1 steht und $R^8$ für Trifluormethyl, Trichlormethyl, Dichlorfluormethyl, Difluorchlormethyl, Dichlormethyl, Chlormethyl, Difluormethyl, Pentafluorethyl, Tetrafluorethyl, Trifluorchlorethyl, Trifluorethyl, Difluordichlorethyl, Trifluordichlorethyl oder Pentachlorethyl stehen, mit der Maßgabe, daß mindestens einer der Reste $R^3$, $R^4$, $R^5$, $R^6$ oder $R^7$ für einen Rest $-(X')_n-R^8$ steht, wobei jedoch nicht gleichzeitig R für Cyano und $R^5$ für Trifluormethyl stehen.

Im einzelnen seien außer den bei den Herstellungsbeispielen genannten Verbindungen, die folgenden Verbindungen der allgemeinen Formel (I) genannt:

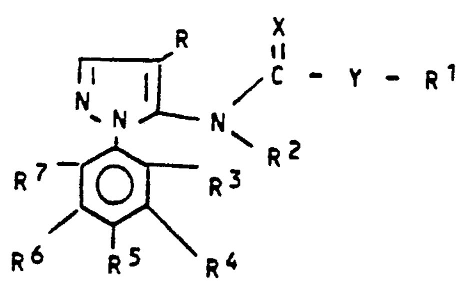

(I)

**Tabelle 1**

| R | $R^1$ | $R^2$ | $R^3$ | $R^4$ | $R^5$ | $R^6$ | $R^7$ | X | Y |
|---|---|---|---|---|---|---|---|---|---|
| CN | $CH_3$ | H | F | H | $OCF_3$ | H | H | O | O |
| CN | $CH_3$ | H | F | H | $OCF_3$ | H | F | O | O |
| CN | $CH_3$ | H | F | F | $OCF_3$ | F | F | O | O |
| CN | $CH_3$ | H | Cl | H | $OCF_3$ | H | H | O | O |
| CN | $CH_3$ | H | Cl | H | $OCF_3$ | H | Cl | O | O |
| CN | $CH_3$ | H | Cl | Cl | $OCF_3$ | H | H | S | O |
| CN | $CH_3$ | H | Cl | H | $OCF_3$ | H | F | O | $N-CH_3$ |
| CN | $CH_3$ | H | Br | H | $OCF_3$ | H | H | O | S |
| CN | $CH_3$ | H | Br | H | $OCF_3$ | H | Br | O | O |
| CN | $C_2H_5$ | F | H | H | $OCF_3$ | H | H | O | $N-CH_3$ |
| CN | $C_2H_5$ | H | F | H | $OCF_3$ | H | F | O | O |
| CN | $C_2H_5$ | H | F | F | $OCF_3$ | F | F | O | $N-CH_3$ |
| CN | $C_2H_5$ | $CH_3$ | Cl | H | $OCF_3$ | H | H | O | O |
| CN | $C_2H_5$ | H | Cl | Cl | $OCF_3$ | H | H | O | $N-CH_3$ |
| CN | $C_2H_5$ | H | Cl | H | $OCF_3$ | H | F | O | O |
| CN | $C_2H_5$ | H | Br | H | $OCF_3$ | H | H | O | S |
| CN | $C_2H_5$ | H | Br | H | $OCF_3$ | H | Br | O | S |
| CN | $C_2H_5$ | $CH_3$ | Cl | H | $OCF_3$ | H | Cl | O | O |
| CN | 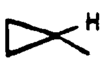 | H | F | H | $OCF_3$ | H | H | O | O |
| CN | 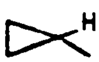 | $CH_3$ | F | H | $OCF_3$ | H | F | O | O |
| CN | 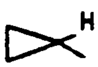 | H | F | F | $OCF_3$ | F | F | O | $N-OCH_3$ |
| CN | 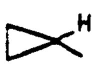 | H | Cl | H | $OCF_3$ | H | H | O | S |
| CN | 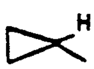 | H | Cl | H | $OCF_3$ | H | Cl | O | O |
| CN | 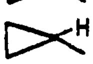 | H | Cl | Cl | $OCF_3$ | H | H | O | O |
| CN | 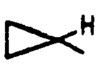 | H | Cl | H | $OCF_3$ | H | F | O | S |
| CN | 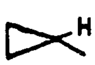 | H | Br | H | $OCF_3$ | H | H | O | $N-CH_3$ |
| CN | 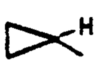 | H | Br | H | $OCF_3$ | H | Br | O | $N-CH_3$ |
| CN | $CH_3$ | H | Cl | H | $SCH_2CF_3$ | H | H | O | S |
| CN | $CH_3$ | H | Cl | H | $SCH_2CF_3$ | H | Cl | O | S |

**Tabelle 1** (Fortsetzung)

| R | $R^1$ | $R^2$ | $R^3$ | $R^4$ | $R^5$ | $R^6$ | $R^7$ | X | Y |
|---|---|---|---|---|---|---|---|---|---|
| CN | $CH_3$ | H | Br | H | $SCH_2CF_3$ | H | H | O | O |
| CN | $CH_3$ | H | Br | H | $SCH_2CF_3$ | H | Br | O | O |
| CN | $CH_3$ | H | Cl | Cl | $SCH_2CF_3$ | H | H | O | O |
| CN | $C_2H_5$ | H | Cl | H | $SCH_2CF_3$ | H | H | O | O |
| CN | $C_2H_5$ | H | Cl | H | $SCH_2CF_3$ | H | Cl | O | O |
| CN | $C_2H_5$ | H | Br | H | $SCH_2CF_3$ | H | H | O | $N-CH_3$ |
| CN | $C_2H_5$ | H | Br | H | $SCH_2CF_3$ | H | Br | O | O |
| CN | $C_2H_5$ | H | Cl | Cl | $SCH_2CF_3$ | H | H | O | O |
| CN | 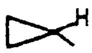 | H | Cl | H | $SCH_2CF_3$ | H | H | O | O |
| CN | 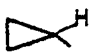 | H | Cl | H | $SCH_2CF_3$ | H | Cl | O | $N-CH_3$ |
| CN | 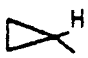 | H | Br | H | $SCH_2CF_3$ | H | H | O | $N-CH_3$ |
| CN | 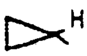 | H | Br | H | $SCH_2CF_3$ | H | Br | O | S |
| CN | 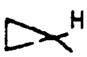 | H | Cl | Cl | $SCH_2CF_3$ | H | H | O | S |
| cn | $CH_3OCH_2$ | H | Cl | H | $SCH_2CF_3$ | H | H | O | S |
| CN | $CH_3OCH_2$ | H | Cl | H | $SCH_2CF_3$ | H | Cl | O | $N-CH_3$ |
| CN | $CH_3OCH_2$ | H | Br | H | $SCH_2CF_3$ | H | H | O | $N-CH_3$ |
| CN | $CH_3OCH_2$ | H | Br | H | $SCH_2CF_3$ | H | Br | O | O |
| CN | $CH_3OCH_2$ | H | Cl | Cl | $SCH_2CF_3$ | H | H | O | O |
| CN | $CH_3$ | H | Cl | H | $OCH_2CF_3$ | H | H | O | O |
| CN | $CH_3$ | H | Cl | H | $OCH_2CF_3$ | H | Cl | O | $N-CH_3$ |
| CN | $CH_3$ | H | Br | H | $OCH_2CF_3$ | H | H | O | O |
| CN | $CH_3$ | H | Br | H | $OCH_2CF_3$ | H | Br | O | O |
| CN | $C_2H_5$ | H | Cl | H | $OCH_2CF_3$ | H | H | O | $N-CH_3$ |
| CN | $C_2H_5$ | H | Cl | H | $OCH_2CF_3$ | H | Cl | O | S |
| CN | $C_2H_5$ | H | Br | H | $OCH_2CF_3$ | H | H | O | S |
| CN | $C_2H_5$ | H | Br | H | $OCH_2OCF_3$ | H | Br | O | $N-CH_3$ |
| CN | 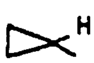 | H | Cl | H | $OCH_2CF_3$ | H | H | O | O |
| CN | 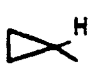 | H | Cl | H | $OCH_2CF_3$ | H | Cl | O | S |
| CN | 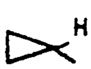 | H | Br | H | $OCH_2CF_3$ | H | H | O | S |

**Tabelle 1** (Fortsetzung)

| R | $R^1$ | $R^2$ | $R^3$ | $R^4$ | $R^5$ | $R^6$ | $R^7$ | X | Y |
|---|---|---|---|---|---|---|---|---|---|
| CN | 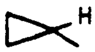 | H | Br | H | $OCH_2CF_3$ | H | Br | O | $N-CH_3$ |
| CN | $CH_3OCH_2-$ | H | Cl | H | $OCH_2CF_3$ | H | H | O | O |
| CN | $CH_3OCH_2-$ | H | Cl | H | $OCH_2CF_3$ | H | Cl | O | O |
| CN | $CH_3OCH_2-$ | H | Br | H | $OCH_2CF_3$ | H | H | O | O |
| CN | $CH_3OCH_2-$ | H | Br | H | $OCH_2CF_3$ | H | Br | O | O |
| CN | $CH_3$ | H | $SCF_3$ | H | Cl | H | H | O | S |
| CN | $C_2H_5$ | H | $SCF_3$ | H | Cl | H | H | O | S |
| CN | $CH_3$ | H | $SCF_3$ | H | Cl | H | Cl | O | $N-CH_3$ |
| CN | $C_2H_5$ | H | $SCF_3$ | H | Cl | H | Cl | O | S |
| CN | $CH_3$ | H | Cl | H | $SO_2CH_2CF_3$ | H | Cl | O | O |
| CN | $CH_3$ | H | Cl | H | $SO_2CH_2CF_3$ | H | Cl | O | $N-CH_3$ |
| CN | $C_2H_5$ | H | Cl | H | $SO_2CH_2CF_3$ | H | Cl | O | O |
| CN | $C_2H_5$ | H | Cl | H | $SO_2CH_2CF_3$ | H | Cl | O | $N-CH_3$ |
| CN | $CH_3$ | H | F | H | $SCF_3$ | H | H | O | O |
| CN | $CH_3$ | H | F | H | $SCF_3$ | H | F | O | O |
| CN | $CH_3$ | H | F | F | $SCF_3$ | F | F | O | O |
| CN | $CH_3$ | H | Cl | H | $SCF_3$ | H | H | O | $N-CH_3$ |
| CN | $CH_3$ | H | Cl | H | $SCF_3$ | H | CL | O | $N-CH_3$ |
| CN | $CH_3$ | H | Cl | Cl | $SCF_3$ | H | H | O | S |
| CN | $CH_3$ | H | Cl | H | $SCF_3$ | H | F | O | S |
| CN | $CH_3$ | H | Br | H | $SCF_3$ | H | H | O | O |
| CN | $CH_3$ | H | Br | H | $SCF_3$ | H | Br | O | O |
| CN | $C_2H_5$ | H | F | H | $SCF_3$ | H | H | O | O |
| CN | $C_2H_5$ | H | F | H | $SCF_3$ | H | F | O | $N-CH_3$ |
| CN | $C_2H_5$ | H | F | H | $SCF_3$ | F | F | O | $N-CH_3$ |

**Tabelle 1** (Fortsetzung)

| R | $R^1$ | $R^2$ | $R^3$ | $R^4$ | $R^5$ | $R^6$ | $R^7$ | X | Y |
|---|---|---|---|---|---|---|---|---|---|
| CN | $C_2H_5$ | H | Cl | H | $SCF_3$ | H | H | O | O |
| CN | $C_2H_5$ | H | Cl | H | $SCF_3$ | H | Cl | O | O |
| CN | $C_2H_5$ | H | Cl | H | $SCF_3$ | H | F | O | O |
| CN | $C_2H_5$ | H | Br | H | $SCF_3$ | H | H | O | S |
| CN | 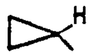 | H | F | H | $SCF_3$ | H | H | O | S |
| CN | 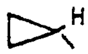 | H | F | H | $SCF_3$ | H | F | O | S |
| CN | 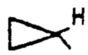 | H | F | F | $SCF_3$ | F | F | O | $N-CH_3$ |
| CN | 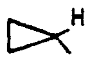 | H | Cl | H | $SCF_3$ | H | H | O | $N-CH_3$ |
| CN | 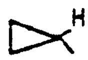 | H | Cl | H | $SCF_3$ | H | Cl | O | O |
| CN | 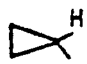 | H | Cl | Cl | $SCF_3$ | H | H | O | O |
| CN | 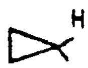 | H | Cl | H | $SCF_3$ | H | F | O | O |
| CN | 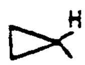 | H | Br | H | $SCF_3$ | H | H | O | S |
| CN | 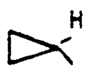 | H | Br | H | $SCF_3$ | H | Br | O | S |
| CN | H | H | $CF_3$ | H | $SO_2CH_3$ | H | H | O | O |
| CN | H | H | $CF_3$ | H | $SO_2CH_3$ | H | H | O | S |
| CN | H | H | $CF_3$ | H | $SCF_3$ | H | H | O | $N-CH_3$ |
| CN | H | H | $OCF_3$ | H | $OCF_3$ | H | H | O | S |
| CN | $CH_3$ | H | $CF_3$ | H | $SO_2CH_3$ | H | H | O | O |
| CN | $CH_3$ | H | $CF_3$ | H | $SO_2CH_3$ | H | H | O | O |
| CN | $CH_3$ | H | $CF_3$ | H | $SCF_3$ | H | H | O | O |
| CN | $CH_3$ | H | $OCF_3$ | H | $OCF_3$ | H | H | O | S |
| CN | $C_2H_5$ | H | $CF_3$ | H | $SO_2CH_3$ | H | H | O | $N-CH_3$ |
| CN | $C_2H_5$ | H | $CF_3$ | H | $SO_2CH_3$ | H | H | O | S |
| CN | $C_2H_5$ | H | $CF_3$ | H | $SCF_3$ | H | H | O | O |
| CN | $C_2H_5$ | H | $OCF_3$ | H | $OCF_3$ | H | H | O | $N-CH_3$ |
| CN | 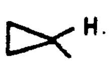 | H | $CF_3$ | H | $SO_2CH_3$ | H | H | O | O |
| CN | 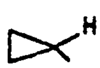 | $CH_3$ | $CF_3$ | H | $SO_2CH_3$ | H | H | O | O |
| CN | 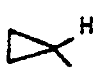 | $C_2H_5$ | $CF_3$ | H | $SCF_3$ | H | H | O | O |
| CN | 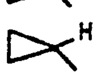 | $C_3H_7$ | $OCF_3$ | H | $OCF_3$ | H | H | O | O |

**Tabelle 1** (Fortsetzung)

| R | $R^1$ | $R^2$ | $R^3$ | $R^4$ | $R^5$ | $R^6$ | $R^7$ | X | Y |
|---|---|---|---|---|---|---|---|---|---|
| CN | H | H | Cl | H | $SO_2CH_2CF_3$ | H | H | O | $N-CH_3$ |
| CN | H | H | Cl | H | $SO_2CH_2CF_3$ | H | H | O | $N-C_2H_5$ |
| CN | $CH_3$ | $CH_3$ | Cl | H | $SCHF_2$ | H | H | O | O |
| CN | $CH_3$ | $C_2H_5$ | Cl | H | $SCH_2F$ | H | Cl | O | O |
| CN | $CH_3$ | $C_3H_7$ | Br | H | $SCHF_2$ | H | H | O | O |
| CN | $CH_3$ | H | Br | H | $SCHF_2$ | H | Br | O | $N-CH_3$ |
| CN | $C_2H_5$ | $CH_3$ | Cl | H | $SCH_2F$ | H | H | O | S |
| CN | $C_2H_5$ | $C_2H_5$ | Cl | H | $SCHF_2$ | H | Cl | O | S |
| CN | $C_2H_5$ | $C_2H_5$ | Br | H | $SCHF_2$ | H | H | O | $N-CH_3$ |
| CN | $C_2H_5$ | $C_3H_7$ | Br | H | $SCHF_2$ | H | Br | O | S |
| CN | 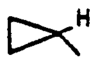 | $C_3H_7$ | Cl | H | $SCHF_2$ | H | H | O | $N-CH_3$ |
| CN | 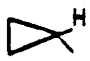 | H | Cl | H | $SCHF_2$ | H | Cl | O | S |
| CN | 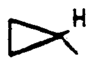 | $CH_3$ | Br | H | $SCHF_2$ | H | H | O | S |
| CN | 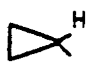 | $C_3H_7$ | Br | H | $SCHF_2$ | H | Br | O | $N-CH_3$ |
| CN | $CH_3$ | H | Cl | H | $SCF_2CHF_2$ | H | H | O | O |
| CN | $CH_3$ | H | Cl | H | $SCF_2CHF_2$ | H | Cl | O | S |
| CN | $CH_3$ | H | Br | H | $SCF_2CHF_2$ | H | H | O | $N-CH_3$ |
| CN | $CH_3$ | $CH_3$ | Br | H | $SCF_2CHF_2$ | H | Br | O | O |
| CN | $C_2H_5$ | $CH_3$ | Cl | H | $SCF_2CHF_2$ | H | H | O | S |
| CN | $C_2H_5$ | $CH_3$ | Cl | H | $SCF_2CHF_2$ | H | Cl | O | $N-CH_3$ |
| CN | $C_2H_5$ | $C_2H_5$ | Br | H | $SCF_2CHF_2$ | H | H | O | O |
| CN | $C_2H_5$ | $C_2H_5$ | Br | H | $SCF_2CHF_2$ | H | Br | O | S |
| CN | 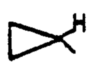 | $C_2H_5$ | Cl | H | $SCF_2CHF_2$ | H | H | O | $N-CH_3$ |
| CN | 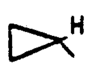 | $C_3H_7$ | Cl | H | $SCF_2CHF_2$ | H | Cl | O | O |
| CN | 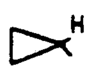 | $C_3H_7$ | Br | H | $SCF_2CHF_2$ | H | H | O | S |
| CN | 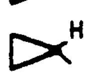 | $C_3H_7$ | Br | H | $SCF_2CHF_2$ | H | Br | O | $N-CH_3$ |
| CN | $CH_3$ | H | Cl | H | $SCF_2CHFCl$ | H | H | O | O |
| CN | $CH_3$ | H | Cl | H | $SCF_2CHFCl$ | H | Cl | O | O |
| CN | $CH_3$ | H | Br | H | $SCF_2CHFCl$ | H | H | O | S |

**Tabelle 1** (Fortsetzung)

| R | $R^1$ | $R^2$ | $R^3$ | $R^4$ | $R^5$ | $R^6$ | $R^7$ | X | Y |
|---|---|---|---|---|---|---|---|---|---|
| CN | $CH_3$ | H | Br | H | $SCF_2CHFCl$ | H | Br | O | O |
| CN | $C_2H_5$ | H | Cl | H | $SCF_2CHFCl$ | H | H | O | S |
| CN | $C_2H_5$ | H | Cl | H | $SCF_2CHFCl$ | H | Cl | O | S |
| CN | $C_2H_5$ | H | Br | H | $SCF_2CHFCl$ | H | H | O | S |
| CN | $C_2H_5$ | H | Br | H | $SCF_2CHFCl$ | H | Br | O | $N-CH_3$ |
| CN | 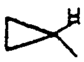 | H | Cl | H | $SCF_2CHFCl$ | H | H | O | O |
| CN | 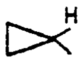 | H | Cl | H | $SCF_2CHFCl$ | H | Cl | O | $N-CH_3$ |
| CN | 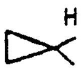 | H | Br | H | $SCF_2CHFCl$ | H | H | O | $N-CH_3$ |
| CN | 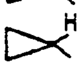 | H | Br | H | $SCF_2CHFCl$ | H | Br | O | $N-CH_3$ |
| CN | $ClCH_2$ | $CH_3$ | Cl | H | $SCF_3$ | H | H | O | $N-CH_3$ |
| CN | $ClCH_2$ | $CH_3$ | Cl | H | $OCF_3$ | H | Cl | O | S |
| CN | $ClCH_2$ | $C_2H_5$ | Br | H | $OCF_3$ | H | H | O | $N-CH_3$ |
| CN | $ClCH_2$ | H | Br | H | $OCF_3$ | H | Br | O | $N-CH_3$ |
| CN | $CH_3OCH_2$ | $C_3H_7$ | Cl | H | $OCF_3$ | H | H | O | S |
| CN | $CH_3OCH_2$ | H | Cl | H | $OCF_3$ | H | Cl | O | O |
| CN | $ClCH_2$ | H | Cl | H | $SCF_3$ | H | H | O | S |
| CN | $ClCH_2$ | H | Cl | H | $SCF_3$ | H | Cl | O | $N-CH_3$ |
| CN | $ClCH_2$ | H | Br | H | $SCF_3$ | H | H | O | S |
| CN | $CH_3$ | $CH_3$ | Cl | H | $S(O)CF_3$ | H | H | O | S |
| CN | $CH_3$ | $CH_3$ | Cl | H | $S(O)CF_3$ | H | Cl | O | O |
| CN | $CH_3$ | $C_3H_7$ | Br | H | $S(O)CF_3$ | H | Br | O | $N-CH_3$ |
| CN | $CH_3$ | $C_2H_5$ | Br | H | $S(O)CF_3$ | H | H | O | O |
| CN | $CH_3$ | $C_3H_7$ | $CF_3$ | H | $S(O)CF_3$ | H | H | O | O |
| CN | $CH_3$ | H | $CF_3$ | H | $S(O)CF_3$ | H | Cl | O | O |
| CN | $C_2H_5$ | H | Cl | H | $S(O)CF_3$ | H | H | O | O |
| CN | $C_2H_5$ | $CH_3$ | Cl | H | $S(O)CF_3$ | H | Cl | O | S |
| CN | $C_2H_5$ | $CH_3$ | Br | H | $S(O)CF_3$ | H | Br | O | $N-CH_3$ |
| CN | $C_2H_5$ | H | Br | H | $S(O)CF_3$ | H | H | O | O |
| CN | $C_2H_5$ | H | $CF_3$ | H | $S(O)CF_3$ | H | H | O | S |
| CN | $C_2H_5$ | H | $CF_3$ | H | $S(O)CF_3$ | H | Cl | O | O |

**Tabelle 1** (Fortsetzung)

| R | $R^1$ | $R^2$ | $R^3$ | $R^4$ | $R^5$ | $R^6$ | $R^7$ | X | Y |
|---|---|---|---|---|---|---|---|---|---|
| CN | 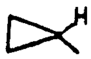 | H | Cl | H | $S(O)CF_3$ | H | H | O | $N-CH_3$ |
| CN | 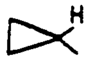 | H | Cl | H | $S(O)CF_3$ | H | Cl | O | O |
| CN | 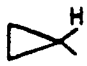 | H | Br | H | $S(O)CF_3$ | H | Br | O | S |
| CN | 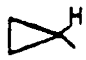 | H | Br | H | $S(O)CF_3$ | H | H | O | S |
| CN | 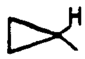 | H | $CF_3$ | H | $S(O)CF_3$ | H | H | O | $N-CH_3$ |
| CN | 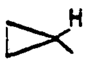 | H | $CF_3$ | H | $S(O)CF_3$ | H | Cl | O | O |
| CN | $CH_3$ | H | Cl | H | $OCF_2CHFCl$ | H | H | O | S |
| CN | $CH_3$ | H | Cl | H | $OCF_2CHFCl$ | H | Cl | O | S |
| CN | $CH_3$ | $CH_3$ | Br | H | $OCF_2CHFCl$ | H | H | O | O |
| CN | $CH_3$ | $C_2H_5$ | Br | H | $OCF_2CHFCl$ | H | Br | O | $N-CH_3$ |
| CN | $C_2H_5$ | $C_3H_7$ | Cl | H | $OCF_2CHFCl$ | H | H | O | O |
| CN | $C_2H_5$ | $C_2H_5$ | Cl | H | $OCF_2CHFCl$ | H | Cl | O | S |
| CN | $C_2H_5$ | $CH_3$ | Br | H | $OCF_2CHFCl$ | H | H | O | $N-CH_3$ |
| CN | $C_2H_5$ | H | Br | H | $OCF_2CHFCl$ | H | Br | O | O |
| CN | 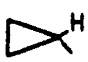 | H | Cl | H | $OCF_2CHFCl$ | H | H | O | S |
| CN | 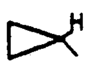 | H | Cl | H | $OCF_2CHFCl$ | H | Cl | O | S |
| CN | 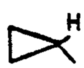 | H | Br | H | $OCF_2CHFCl$ | H | H | N | $N-CH_3$ |
| CN | 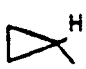 | H | Br | H | $OCF_2CHFCl$ | H | Br | O | $N-CH_3$ |
| CN | $CH_3$ | $CH_3$ | Cl | H | $OCF_2CHCl_2$ | H | H | O | O |
| CN | $CH_3$ | $C_2H_5$ | Cl | H | $OCF_2CHCl_2$ | H | Cl | O | O |
| CN | $CH_3$ | $C_3H_7$ | Br | H | $OCF_2CHCl_2$ | H | H | O | O |
| CN | $CH_3$ | H | Br | H | $OCF_2CHCl_2$ | H | Br | O | O |
| CN | $C_2H_5$ | H | Cl | H | $OCF_2CHCl_2$ | H | H | O | O |
| CN | $C_2H_5$ | H | Cl | H | $OCF_2CHCl_2$ | H | Cl | O | S |
| CN | $C_2H_5$ | H | Br | H | $OCF_2CHCl_2$ | H | H | O | $N-CH_3$ |
| CN | $C_2H_5$ | $CH_3$ | Br | H | $OCF_2CHCl_2$ | H | Br | O | O |

**Tabelle 1** (Fortsetzung)

| R | $R^1$ | $R^2$ | $R^3$ | $R^4$ | $R^5$ | $R^6$ | $R^7$ | X | Y |
|---|---|---|---|---|---|---|---|---|---|
| CN | 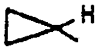 | $C_2H_5$ | Cl | H | $OCF_2CHCl_2$ | H | H | O | O |
| CN | 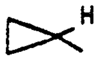 | $C_3H_7$ | Cl | H | $OCF_2CHCl_2$ | H | Cl | O | O |
| CN | 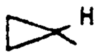 | H | Br | H | $OCF_2CHCl_2$ | H | H | O | O |
| CN | 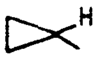 | $CH_3$ | Br | H | $OCF_2CHCl_2$ | H | Br | O | S |
| CN | $CH_3$ | $C_2H_5$ | Cl | H | $OCF_2CHF_2$ | H | H | O | S |
| CN | $CH_3$ | $C_3H_7$ | Cl | H | $OCF_2CHF_2$ | H | Cl | O | $N-CH_3$ |
| CN | $CH_3$ | H | Br | H | $OCF_2CHF_2$ | H | H | O | S |
| CN | $CH_3$ | H | Br | H | $OCF_2CHF_2$ | H | Br | O | O |
| CN | $C_2H_5$ | H | Cl | H | $OCF_2CHF_2$ | H | H | O | $N-CH_3$ |
| CN | $C_2H_5$ | H | Cl | H | $OCF_2CHF_2$ | H | Cl | O | S |
| CN | $C_2H_5$ | H | Br | H | $OCF_2CHF_2$ | H | H | O | O |
| CN | $C_2H_5$ | H | Br | H | $OCF_2CHF_2$ | H | Br | O | S |
| CN | 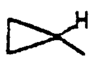 | H | Cl | H | $OCF_2CHF_2$ | H | H | O | S |
| CN | 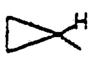 | H | Cl | H | $OCF_2CHF_2$ | H | Cl | O | S |
| CN | 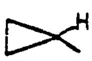 | H | Br | H | $OCF_2CHF_2$ | H | H | O | $N-CH_3$ |
| CN | 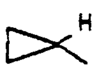 | H | Br | H | $OCF_2CHF_2$ | H | Br | O | $N-CH_3$ |
| CN | $CH_3$ | H | Cl | H | $SO_2CH_3$ | H | H | O | O |
| CN | $CH_3$ | H | Cl | H | $SO_2CH_3$ | H | Cl | O | S |
| CN | $CH_3$ | H | Br | H | $SO_2CH_3$ | H | H | O | $N-CH_3$ |
| CN | $CH_3$ | $CH_3$ | Br | H | $SO_2CH_3$ | H | Br | O | O |
| CN | $CH_3$ | $CH_3$ | $CF_3$ | H | $SO_2CH_3$ | H | H | O | S |
| CN | $C_2H_5$ | $CH_3$ | Cl | H | $SO_2CH_3$ | H | H | O | $N-CH_3$ |
| CN | $C_2H_5$ | $C_2H_5$ | Cl | H | $SO_2CH_3$ | H | Cl | O | O |
| CN | $C_2H_5$ | $C_2H_5$ | Br | H | $SO_2CF_3$ | H | H | O | S |
| CN | $C_2H_5$ | $C_2H_5$ | Br | H | $SO_2CF_3$ | H | Br | O | O |
| CN | $C_2H_5$ | $CH_3$ | $CF_3$ | H | $SO_2CF_3$ | H | H | O | $N-CH_3$ |
| CN | 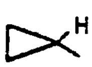 | $CH_3$ | Cl | H | $SO_2CF_3$ | H | H | O | S |
| CN | 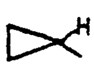 | $CH_3$ | Cl | H | $SO_2CF_3$ | H | Cl | O | O |
| CN | 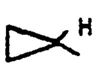 | H | Br | H | $SO_2CF_3$ | H | H | O | $N-CH_3$ |

**Tabelle 1** (Fortsetzung)

| R | $R^1$ | $R^2$ | $R^3$ | $R^4$ | $R^5$ | $R^6$ | $R^7$ | X | Y |
|---|---|---|---|---|---|---|---|---|---|
| CN | 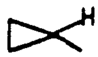 | H | Br | H | $SO_2CF_3$ | H | Br | O | S |
| CN | 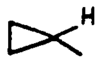 | H | $CF_3$ | H | $SO_2CF_3$ | H | H | O | O |
| CN | $CH_3$ | H | F | H | $SCCl_2F$ | H | H | O | O |
| CN | $CH_3$ | H | F | H | $SCCl_2F$ | H | F | O | S |
| CN | $CH_3$ | H | F | F | $SCCl_2F$ | F | F | O | $N-CH_3$ |
| CN | $CH_3$ | $CH_3$ | Cl | H | $SCCl_2F$ | H | H | O | O |
| CN | $CH_3$ | $CH_3$ | Cl | H | $SCCl_2F$ | H | Cl | O | S |
| CN | $CH_3$ | $CH_3$ | Cl | H | $SCCl_2F$ | H | F | O | $N-CH_3$ |
| CN | $CH_3$ | $C_2H_5$ | Br | H | $SCCl_2F$ | H | H | O | O |
| CN | $CH_3$ | $C_2H_5$ | Br | H | $SCCl_2F$ | H | Br | O | S |
| CN | $C_2H_5$ | $C_2H_5$ | F | H | $SCCl_2F$ | H | H | O | S |
| CN | $C_2H_5$ | $C_2H_5$ | F | H | $SCCl_2F$ | H | F | O | O |
| CN | $C_2H_5$ | $C_2H_5$ | F | H | $SCCl_2F$ | F | F | O | $N-CH_3$ |
| CN | $C_2H_5$ | $CH_3$ | Cl | H | $SCCl_2F$ | H | H | O | S |
| CN | $C_2H_5$ | $CH_3$ | Cl | H | $SCCl_2F$ | H | Cl | O | O |
| CN | $C_2H_5$ | H | Cl | H | $SCCl_2F$ | H | F | O | $N-CH_3$ |
| CN | $C_2H_5$ | H | Br | H | $SCCl_2F$ | H | H | O | S |
| CN | $C_2H_5$ | H | Br | H | $SCCl_2F$ | H | Br | O | O |
| CN | 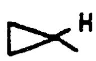 | H | F | H | $SCCl_2F$ | H | H | O | S |
| CN | 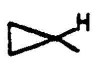 | H | F | H | $SCCl_2F$ | H | F | O | $N-CH_3$ |
| CN | 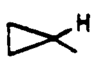 | $CH_3$ | F | F | $SCCl_2F$ | F | F | O | O |
| CN | 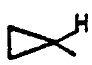 | $CH_3$ | Cl | H | $SCCl_2F$ | H | H | O | S |
| CN | 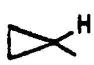 | $CH_3$ | Cl | H | $SCCl_2F$ | H | Cl | O | $N-CH_3$ |
| CN | 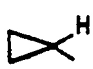 | $C_2H_5$ | Cl | H | $SCCl_2F$ | H | F | O | O |
| CN | 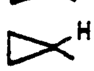 | $C_2H_5$ | Br | H | $SCCl_2F$ | H | H | O | S |
| CN | 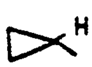 | $C_2H_5$ | Br | H | $SCCl_2F$ | H | Br | O | $N-CH_3$ |
| CN | $CH_3$ | H | F | H | $OCHF_2$ | H | H | O | O |
| CN | $CH_3$ | H | F | H | $OCHF_2$ | H | F | O | S |
| CN | $CH_3$ | H | F | F | $OCHF_2$ | F | F | O | $N-CH_3$ |

**Tabelle 1** (Fortsetzung)

| R | $R^1$ | $R^2$ | $R^3$ | $R^4$ | $R^5$ | $R^6$ | $R^7$ | X | Y |
|---|---|---|---|---|---|---|---|---|---|
| CN | $CH_3$ | H | Cl | H | $OCHF_2$ | H | H | O | O |
| CN | $CH_3$ | H | Cl | H | $OCHF_2$ | H | Cl | O | O |
| CN | $CH_3$ | H | Cl | H | $OCHF_2$ | H | F | O | O |
| CN | $CH_3$ | H | Br | H | $OCHF_2$ | H | H | O | S |
| CN | $CH_3$ | H | Br | H | $OCHF_2$ | H | Br | O | S |
| CN | $C_2H_5$ | H | F | H | $OCHF_2$ | H | H | O | S |
| CN | $C_2H_5$ | H | F | H | $OCHF_2$ | H | F | O | S |
| CN | $C_2H_5$ | H | F | F | $OCHF_2$ | F | F | O | $N-CH_3$ |
| CN | $C_2H_5$ | H | Cl | H | $OCHF_2$ | H | H | O | $N-CH_3$ |
| CN | $C_2H_5$ | H | Cl | H | $OCHF_2$ | H | Cl | O | $N-CH_3$ |
| CN | $C_2H_5$ | H | Cl | H | $OCHF_2$ | H | F | O | $N-CH_3$ |
| CN | $C_2H_5$ | H | Br | H | $OCHF_2$ | H | F | O | O |
| CN | $C_2H_5$ | H | Br | H | $OCHF_2$ | H | Br | O | O |
| CN | 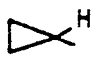 | H | F | H | $OCHF_2$ | H | H | O | O |
| CN | 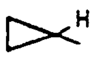 | H | F | H | $OCHF_2$ | H | F | O | S |
| CN | 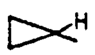 | H | F | F | $OCHF_2$ | F | F | O | O |
| CN | 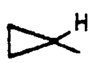 | $CH_3$ | Cl | H | $OCHF_2$ | H | H | O | O |
| CN | 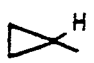 | $CH_3$ | Cl | H | $OCHF_2$ | H | Cl | O | O |
| CN | 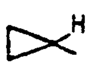 | $CH_3$ | Cl | H | $OCH_2F$ | H | F | O | O |
| CN | 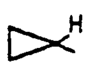 | $C_2H_5$ | Br | H | $OCH_2F$ | H | H | O | O |
| CN | 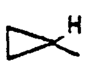 | $C_2H_5$ | Br | H | $OCHF_2$ | H | Br | O | O |
| CN | 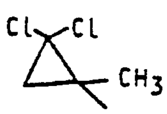 | $C_2H_5$ | Cl | H | $OCF_3$ | H | H | O | $N-CH_3$ |
| CN | 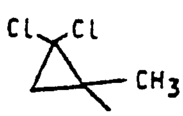 | $C_2H_5$ | Cl | H | $OCF_3$ | H | Cl | O | $N-CH_3$ |
| CN | 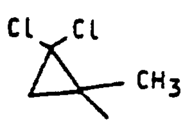 | $C_2H_5$ | Cl | H | $SCF_3$ | H | H | O | $N-CH_3$ |
| CN | 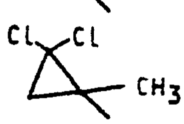 | $C_3H_7$ | Cl | H | $SCF_3$ | H | Cl | O | $N-CH_3$ |

**Tabelle 1** (Fortsetzung)

| R | $R^1$ | $R^2$ | $R^3$ | $R^4$ | $R^5$ | $R^6$ | $R^7$ | X | Y |
|---|---|---|---|---|---|---|---|---|---|
| CN | 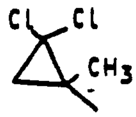 | $C_3H_7$ | Cl | H | $SO_2CF_3$ | H | H | O | $N-CH_3$ |
| CN | 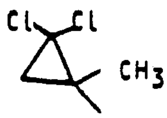 | H | Cl | H | $SO_2CF_3$ | H | Cl | O | $N-C_2H_5$ |
| CN | 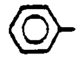 | H | Cl | H | $OCF_3$ | H | Cl | O | $N-C_2H_5$ |
| CN | 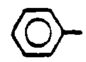 | H | Cl | H | $SCF_3$ | H | Cl | O | $N-C_3H_7$ |
| CN | 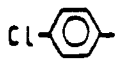 | H | Cl | H | $OCF_3$ | H | Cl | O | $N-CH=CH_2$ |
| CN | 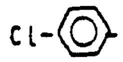 | H | Cl | H | $SCF_3$ | H | Cl | O | $N-C_3H_7$ |
| CN | 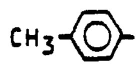 | H | Cl | H | $OCF_3$ | H | Cl | O | $N-C_2H_5$ |
| CN | 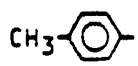 | H | Cl | H | $SCF_3$ | H | Cl | O | $N-CH_3$ |
| CN | 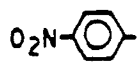 | H | Cl | H | $OCF_3$ | H | Cl | O | $N-C_2H_5$ |
| CN | 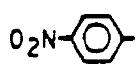 | H | Cl | H | $SCF_3$ | H | Cl | O | $N-C_3H_7$ |

Verwendet man als Ausgangsstoffe beispielsweise 5-Amino-4-cyano-1-(2',6'-dichlor-4'-trifluormethylthiophenyl)-pyrazol und Chlorameisensäurrephenylester, so läßt sich der Reaktionsablauf des erfidungsgemäßen Verfahrens (a - α) durch das folgende Formelschema darstellen:

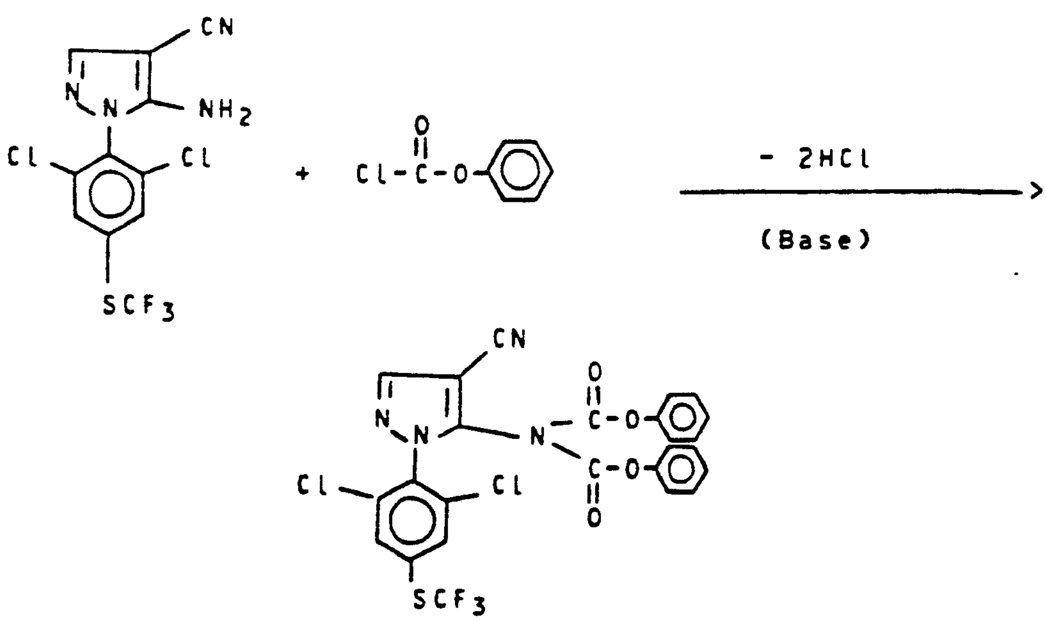


Verwendet man als Ausgangsstoffe beispielsweise 5-Amino-4-methoxycarbonyl-1-(2',6'-dichlor-4-trifluormethoxyphenyl)-pyrazol und Methylisocyanat als Ausgangsstoffe, so läßt sich der Reaktionsablauf des erfindungsgemäßen Verfahrens (a - β) durch das folgende Formelschema darstellen:

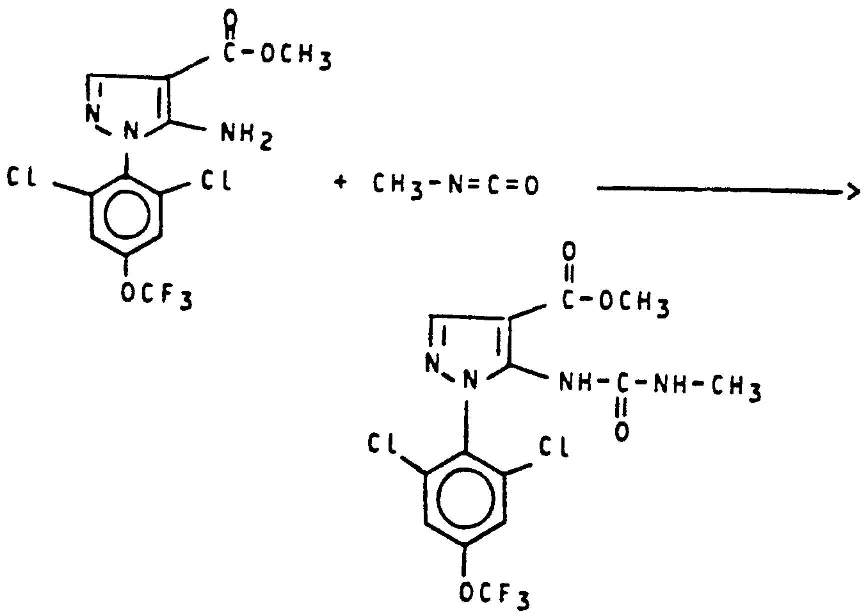

Verwendet man als Ausgangsstoffe beispielsweise 4-Cyano-5-(bisphenoxycarbonylamino)-1-(2',6'-dichlor-4'-trifluormethylthiophenyl)-pyrazol und Methanol, so läßt sich der Reaktionsablauf des erfindungsgemäßen Verfahrens (b) durch das folgende Formelschema darstellen:

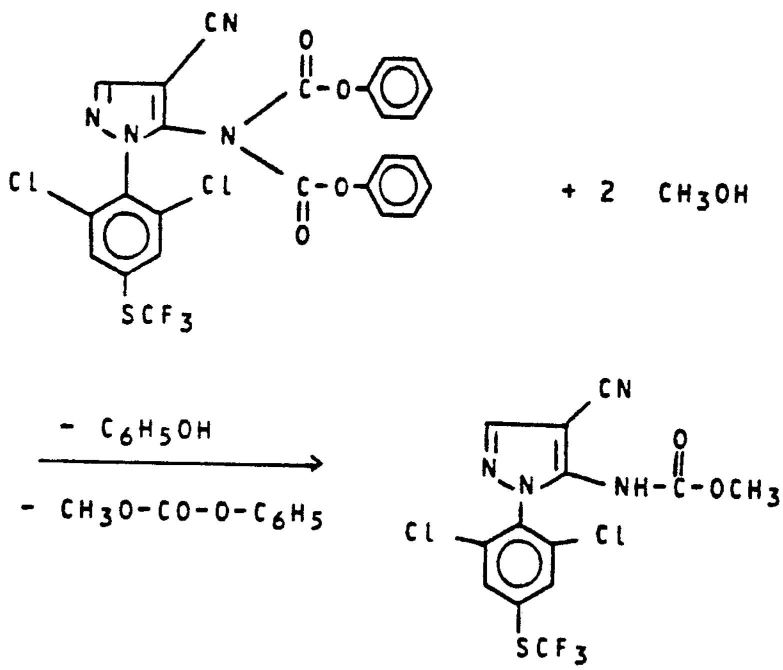


Die zur Durchführung des erfindungsgemäßen Verfahrens (a/α und β) als Ausgangsstofe benötigten 5-Aminopyrazole sind durch die Formel (II) allgemein definiert. In dieser Formel (II) besitzen R, $R^3$, $R^4$, $R^5$, $R^6$ und $R^7$ vorzugsweise diejenige Bedeutung, die schon bei der Beschreibung der erfindungsgemäßen Stoffe der Formel (I) als bevorzugt für diese Substituenten genannt wurden.

Die 5-Aminopyrazole der Formel (II) sind teilweise bekannt (vgl. z. B. EP-34 945: DE-OS-3 226 496 und DE-OS-3 129 429).

Noch nicht bekannt sind 5-Aminopyrazole der Formel (IIb),

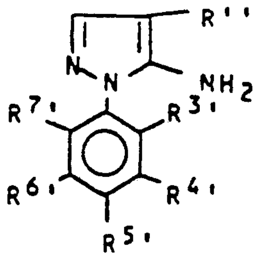

(IIb)

in welcher
R'' für Cyano steht und
$R^{3'}$, $R^{4'}$, $R^{5'}$, $R^{6'}$ und $R^{7'}$, welche gleich oder verschieden sind, für Wasserstoff, Fluor, Chlor, Brom, Iod, Nitro, jeweils geradkettiges oder verzweigtes Alkyl, Alkoxy, Alkylsulfonyl oder Alkoxycarbonyl mit bis zu 4 Kohlenstoffatomen in den jeweiligen Alkylteilen oder für den Rest -X'-$R^8$ stehen, wobei X' für Sauerstoff, Schwefel, Sulfinyl oder Sulfonyl steht und $R^8$ für geradkettiges oder verzweigtes Halogenalkyl mit bis zu 4 Kohlenstoffatomen und bis zu 9 gleichen oder verschiedenen Halogenatomen steht mit der Maßgabe, daß mindestens einer der Reste $R^{3'}$, $R^{4'}$, $R^{5'}$, $R^{6'}$ oder $R^{7'}$ für einen Rest -X'-$R^8$ steht, wobei nicht gleichzeitig X' für Sauerstoff und $R^8$ für Trifluormethyl stehen.
Man erhält sie nach prinzipiell bekannten Verfahren, wenn man Acrylnitril-Derivate der Formel (VIb)

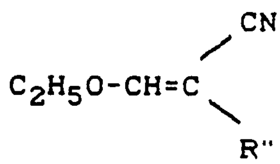

(VIb)

in welcher
R'' die oben angegebene Bedeutung hat,
mit Phenylhydrazinen der Formel (VIa)

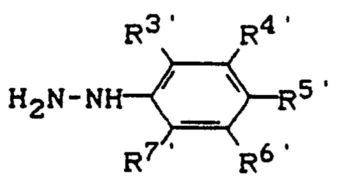

(VIIa)

in welcher
$R^{3'}$, $R^{4'}$, $R^{5'}$, $R^{6'}$ und $R^{7'}$ die oben angegebene Bedeutung haben,
entweder zunächst in einer ersten Stufe, gegebenenfalls in Gegenwart eines Verdünnungsmittels wie beispielsweise Eisessig oder Ethanol, und gegebenenfalls in Gegenwart eines Reaktionshilfsmittels, wie beispielsweise Natriumacetat, bei Temperaturen zwischen -20°C und +20°C umsetzt zu den Phenylhydrazin-Derivaten der Formel (VIIIb),

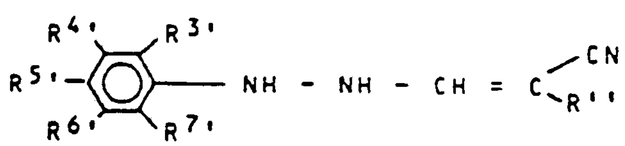

(VIIIb)

in welcher
R'', $R^{3'}$, $R^{4'}$, $R^{5'}$, $R^{6'}$ und $R^{7'}$ die oben angegebene Bedeutung haben,
und diese in einer 2. Stufe, gegebenenfalls in Gegenwart eines Verdünnungsmittels, wie beispielsweise Ethylenglykolmonoethylether, bei Temperaturen zwischen +50°C und +150°C cyclisiert oder in einem Reaktionsschritt ohne Isolierung der Zwischenstufe der Formel (VIIIb), gegebenenfalls in Gegenwart eines Verdünnungsmittels, wie beispielsweise Ethylenglykolmonoethylether oder Ethanol, bei Temperaturen zwischen +50°C und +150°C direkt cyclisiert.
Die Verbindungen der Formeln (IIb), (VIIIa) und (VIIIb) sind Gegenstand der oben genannten älteren Anmeldung (DE-P-3 337 543.7 vom 15.10.1983).
Die Acrylnitril-Derivate der Formel (VIb) sind bekannt (vgl. z. B. EP-34 945 oder DE-OS-3 129 429).
Die Phenylhydrazine der Formel (VIIa) sind größtenteils bekannt oder können nach bekannten Verfahren in einfacher, analoger Art und Weise hergestellt werden (vgl. z. B. Houben-Weyl, "Methoden der organischen

Chemie", Band X/2, S. 203, Thieme Verlag Stuttgart 1967),
indem man beispielsweise die bekannten Aniline der Formel (IX),

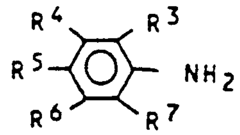

(IX)

in welcher
$R^3$, $R^4$, $R^5$, $R^6$ und $R^7$ die oben angegebene Bedeutung haben,
mit Natriumnitrit in Gegenwart einer Säure, wie beispielsweise Schwefelsäure, und dann mit Zinn-II-chlorid ebenfalls in Gegenwart einer Säure, wie beispielsweise Salzsäure, bei Temperaturen zwischen -20°C und +80°C umsetzt.

Die weiterhin zur Durchführung des erfindungsgemäßen Verfahrens (a - α) als Ausgangsstoffe benötigten Acylierungsmittel sind durch die Formel (III) allgemein definiert. In dieser Formel (III) stehen $R^1$, X und Y vorzugsweise für diejenigen Reste, die schon bei der Beschreibung der erfindungsgemäßen Stoffe der Formel (I) als bevorzugt für diese Substituenten genannt wurden. A steht vorzugsweise für Chlor oder Brom. Die Acylierungsmittel der Formel (III) sind allgemein bekannte Verbindungen der organischen Chemie.

Die zur Durchführung des erfindungsgemäßen Verfahrens (a - β) weiterhin als Ausgangsstoffe benötigten Iso(thio)cyanate sind durch die Formel (IV) allgemein definiert. In dieser Formel (IV) stehen $R^1$ und X vorzugsweise für diejenigen Reste, die schon bei der Beschreibung der erfindungsgemäßen Stoffe der Formel (I) als bevorzugt für diese Substituenten genannt wurden.

Die Iso(thio)cyanate der Formel (IV) sind ebenfalls allgemein bekannte Verbindungen der organischen Chemie.

Die zur Durchführung des erfindungsgemäßen Verfahrens (b) als Ausgangsstoffe benötigten Biscarbamate sind durch die Formel (Ia) allgemein definiert. In dieser Formel (Ia) stehen R, $R^3$, $R^4$, $R^5$, $R^6$ und $R^7$ vorzugsweise für diejenigen Reste, die bereits bei der Beschreibung der erfindungsgemäßen Stoffe der Formel (I) für diese Substituenten genannt wurden.

Die Biscarbamate der Formel (Ia) sind erfindungsgemäße Verbindungen und erhältlich nach Verfahren (a - α).

Die zur Durchführung des erfindungsgemäßen Verfahrens (b) weiterhin als Ausgangsstoffe benötigten Alkohole, Amine oder Thiole sind durch die Formel (V) allgemein definiert. In dieser Formel (V) stehen $R^1$ und Y vorzugsweise für diejenigen Reste, die bereits bei der Beschreibung der erfindungsgemäßen Stoffe der Formel (I) als bevorzugt für diese Substituenten genannt wurden.

Die Alkohole, Amine und Thiole der Formel (V) sind allgemein bekannte Verbindungen der organischen Chemie.

Als Verdünnungsmittel zur Durchführung der erfindungsgemäßen Verfahren (a/α + β) kommen inerte organische Lösungsmittel in Frage. Vorzugsweise erwendet man aliphatische oder aromatische, gegebenenfalls halogenierte Kohlenwasserstoffe, wie Benzin, Benzol, Toluol, Xylol, Pentan, Hexan, Heptan, Cyclohexan, Petrolether, Ligroin, Methylenchlorid, Ethylenchlorid, Chloroform, Tetrachlorkohlenstoff, Chlorbenzol oder Dichlorbenzol, Ether, wie Diethylether oder Diisopropylether, Ethylenglykoldimethylether, Tetrahydrofuran oder Dioxan, Ketone, wie Aceton oder Butanon, Methylisopropylketon oder Methylisobutylketon, Ester, wie Essigsäureethylester, Nitrile, wie Acetonitril oder Propionitril, Amide, wie Dimethylformamid, Dimethylacetamid, N-Methylpyrrolidon oder Hexamethylphosphorsäuretriamid.

Verwendet man Acylierungsmittel der Formel (III) in flüssiger Form, so ist es auch möglich, diese in entsprechendem Überschuß als Verdünnungsmittel einzusetzen.

Als Säurebindemittel oder Katalysatoren kommen für die erfindungsgemäßen Verfahren (a/α + β) alle üblicherweise verwendbaren anorganischen und organischen Basen in Frage. Vorzugsweise verwendet man Alkalimetallhydroxide oder -carbonate, wie beispielsweise Natriumhydroxid, Kaliumhydroxid, Natriumcarbonat oder Kaliumcarbonat oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei den erfindungsgemäßen Verfahren (a/α+ β) in einem größeren Bereich variiert werden. Im allgemeinen arbeitet man zwischen -20°C und +150°C, vorzugsweise zwischen 0°C und +100°C.

Zur Durchführung des erfindungsgemäßen Verfahrens (a) setzt man Pro Mol 5-Aminopyrazol der Formel (II) im allgemeinen 1 bis 20 Mol, vorzugsweise 1 bis 15 Mol an Acylierungsmittel der Formel (III) bzw. Iso(thio)cyanat der Formel (IV) und gegebenenfalls im allgemeinem 1 bis 3 Mol, vorzugsweise 1 bis 2 Mol an Säurebindemittel ein. Die Reaktionsführung, Aufarbeitung und Isolierung der Endprodukte der Formel (I) erfolgt in üblicher Art und weise.

Als Verdünnungsmittel zur Durchführung des erfindumgsgemäßen Verfahrens (b) kommen ebenfalls inerte organische Lösungsmittel in Frage. Vorzugsweise verwendet man die bei Verfahren (a) genannten organischen Lösungsmittel oder Alkohole, wie Methanol, Ethanol oder Isopropanol.

Es ist jedoch auch möglich, die als Reaktionskomponenten verwendeten Alkohole, Amine oder Thiole der Formel (V) in entsprechendem Überschuß gleichzeitig als Verdünnungsmittel einzusetzen.

Die Reaktionstemperaturen können bei dem erfindungsgegemäßen Verfahren (b) ebenfalls in einem größeren Bereich variiert werde. Im allgemeinen arbeitet man zwischen 0°C und +200°C, vorzugsweise zwischen +20°C und +150°C.

Zur Durchführung des erfindungsgemäßen Verfahrems (b) setzt man pro Mol Biscarbamat der Formel (Ia) im allgemeinen 1 bis 20 Mol, vorzugsweise 1 bis 10 Mol Alkohol bzw. Amin oder Thiol der Formel (V) ein und erwärmt für mehrere Stunden auf die erforderliche Temperatur. Die Aufarbeitung und Isolierung der Reaktionsprodukte der Formel (I) erfolgt nach üblichen Verfahren.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z. B. bei den folgenden Pflanzen verwendet werden:

**Dikotyle Unkräuter der Gattungen:** Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

**Dikotyle Kulturen der Gattungen:** Gossypium, Glycine, Beta, Daucus, Phaseolus, Pisum, Solanum, Linun, Ipomoea, Vicia, Nicotiana, Lycopersicon, Arachis, Brassica, Lactuca, Cucumis, Cucurbita.

**Monokotyle Unkräuter der Gattungen:** Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

**Monokotyle Kulturen der Gattungen:** Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z. B. auf Industrie- und Gleisanlagen und auf wegen und Plätzen mit und ohne Baumbewuchs. Ebenso können die Verbindungen zur Unkrautbekämpfung in Dauerkulturen, z. B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei zeigen die erfindungsgemäßen Wirkstoffe der Formel (I) neben einer besonders guten allgemeinen herbiziden Wirksamkeit auch eine deutlich verbesserte Kulturpflanzenselektivität in wichtigen Kulturen, wie beispielsweise Weizen. Die Vorprodukte der Formel (II) ebenso wie die Vorprodukte der Formeln (VIIIa) und (VIIIb) besitzen ebenfalls herbizide Wirkungen und ausgeprägte Selektivität gegenüber wichtigen Kulturpflanzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z. B. auch organische Lösungsmittel als Hilfslösungsmittel verwendet werden. Als flüssige Lösungsmittel kommen im wesentlichen in Frage; Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z. B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage; z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z. B. nichtionogene und anionische Emulgatoren, wie Polyoxyethylen-Fettsäure-Ester, Polyoxyethylen-Fettalkohol-Ether, z. B. Alkylaryl-polyglykolether, Alkylsulfonate, Alkylsulfate, Arylsulfonate sowie Eiweißhydrolysate; als Dispergiermittel kommen in Frage: z.

B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich sind.

Für die Mischungen kommen bekannte Herbizide, beispielsweise Benzonitrile, Diphenylether, Pyridoxyphenoxypropionsäuren, Phenoxyalkancarbonsäuren, Harnstoffe, Triazinone oder Triazindione, wie z. B. 3,5-Dibrom- oder 3,5-Diiod-4-hydroxy-benzonitril, α-[4-(3,5-Dichlor-2-pyridoxy)-phenoxy]-propionsäure-2-benzyloxyethylester, -2,2-diethoxyethylester oder -trimethylsilylmethylester, 2,4-Dichlorphenoxyessigsäure, α-(2,4-Dichlorphenoxy)-propionsäure, 4-Chlor-2-methylphenoxy-essigsäure, α-(4-Chlor-2-methylphenoxy)-propionsäure, 1-Amino-6-ethylthio-3-(2,2-dimethylpropyl)-1,3,5-triazin-2,4-(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio- oder -3-ethylthio-1,2,4-triazin-5(4H)-on zur Unkrautbekämpfung in Sojabohnen in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einer größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

**Herstellungsbeispiel**

**Beispiel 1**

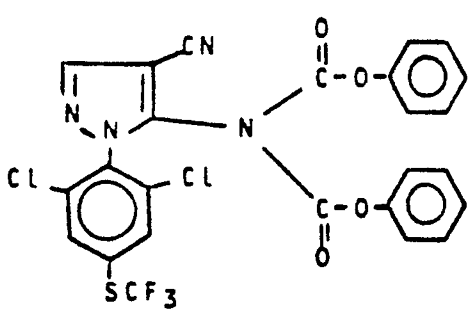

(Verfahren a - α)

35,3 g (0,1 Mol) 5-Amino-4-cyano-1-(2,6-dichlor-4-trifluormethylthiophenyl)-pyrazol in 100 ml Chloroform werden unter Rühren und Eiskühlung bei 0°C bis 5°C tropfenweise zunächst mit 52,2 g (0,3 Mol) Chlorameisensäurephenylester und danach mit 23,7 g (0,3 Mol) Pyridin in 30 ml Chloroform versetzt. Nach beendeter Zugabe rührt man weitere 16 Stunden bei Raumtemperatur, saugt den Feststoff ab, wäscht mit Chloroform nach und verrührt den Rückstand mit Wasser. Das kristalline Produkt wird abgesaugt, getrocknet und aus Toluol umkristallisiert. Man erhält 46 g (78 % der Theorie) an 4-Cyano-5-(N,N-bisphenoxycarbonylamino)-1-(2,6-dichlor-4-trifluormethylthio)-pyrazol vom Schmelzpunkt 164 bis 167° C.

**Beispiel 2**

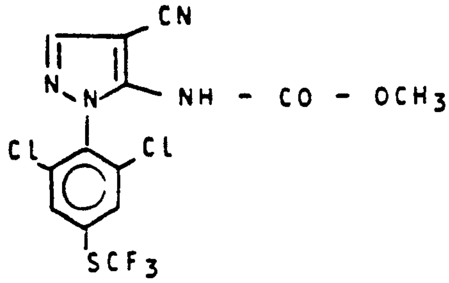

(Verfahren b)

18,2 g (0,02 Mol) 4-Cyano-5-(N,N-bisphenoxycarbonylamino)-1-(2,6-dichlor-4-trifluormethylthio)-pyrazol werden in 200 ml (160 g, 5 Mol) wasserfreiem Methanol zum Sieden erhitzt und so lange unter Rückfluß gekocht, bis eine klare Lösung entstanden ist. Das überschüssige Methanol wird abdestilliert, der Rückstand in Chloroform aufgenommen, mit wäßriger Natriumhydrogencarbonatlösung gewaschen, über Natriumsulfat getrocknet, eingeengt und aus Toluol umkristallisiert. Man erhält 5,8 g (71 % der Theorie) an 4-Cyano-1-(2,6-dichlor-4-trifluormethylthio-phenyl)-5-methoxycarbonylamino-pyrazol vom Schmelzpunkt 145° C.

In entsprechender Weise und gemäß den allgemeinen Herstellungsangaben erhält man die folgenden substituierten 5-Acylamino-1-phenylpyrazole der Formel (I):

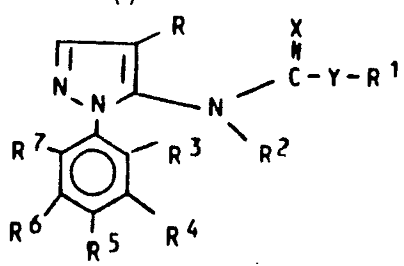

(I)

**Tabelle 2**

| Bsp. Nr. | R | $R^1$ | $R^2$ | $R^3$ | $R^4$ | $R^5$ | $R^6$ | $R^7$ | X | Y | physikalische Eigenschaften |
|---|---|---|---|---|---|---|---|---|---|---|---|
| 3 | CN | $CH_3$ | H | Cl | H | $-OCF_3$ | H | H | O | O | Fp: 122° C |
| 4 | CN | $CH_3$ | H | Cl | H | $-OCF_3$ | H | H | O | $-\underset{CH_3}{\underset{\vert}{N}}-$ | Fp: 140° C |
| 5 | CN | $C_6H_5$ | $C_6H_5-\overset{O}{\overset{\Vert}{C}}-$ | Cl | H | $-OCF_3$ | H | H | O | O | Fp: 155 - 157° C |
| 6 | CN | $C_2H_5$ | H | Cl | H | $-OCF_3$ | H | H | O | O | Fp: 90° C |
| 7 | CN | $C_6H_5$ | $C_6H_5-\overset{O}{\overset{\Vert}{C}}-$ | Cl | H | $-OCF_3$ | H | Cl | O | O | Fp: 150° C |
| 8 | CN | $CH_3$ | H | Cl | H | $-OCF_3$ | H | Cl | O | O | Fp: 108 - 110° C |
| 9 | CN | $C_6H_5$ | $C_6H_5-\overset{O}{\overset{\Vert}{C}}-$ | Cl | H | $CF_3SO_2-$ | H | Cl | O | O | Fp: 155 - 157° C |
| 10 | CN | $CH_3$ | H | Cl | H | $CF_3SO_2-$ | H | Cl | O | $-\underset{CH_3}{\underset{\vert}{N}}-$ | Fp: 189 - 190° C |
| 11 | CN | $CH_3$ | H | Cl | H | $-OCF_3$ | H | Cl | O | $-\underset{CH_3}{\underset{\vert}{N}}-$ | Fp: 172 - 173° C |
| 12 | CN | $CH_3$ | H | Cl | H | $CF_3SO_2-$ | H | Cl | O | O | Fp: 198 - 200° C |
| 13 | CN | $CH_3$ | H | Cl | H | $-SCF_3$ | H | Cl | O | $-\underset{CH_3}{\underset{\vert}{N}}-$ | Fp: 198 - 199° C |
| 14 | CN | $CH_3$ | H | Cl | H | $-CF_3$ | H | Cl | O | $-\underset{CH_3}{\underset{\vert}{N}}-$ | Fp: 224 - 225° C |
| 15 | CN | $C_2H_5$ | H | Cl | H | $-SO_2CF_3$ | H | Cl | O | O | Fp: 182° C |
| 16 | CN | $n-C_4H_9$ | H | Cl | H | $-SO_2CF_3$ | H | Cl | O | S | Fp: 147° C |

**Herstellung der Ausgangsstoffe:**

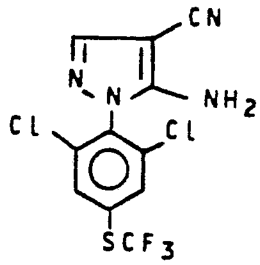

(II-1)

14,1 g (0,04 Mol) 1-(2,2-Dicyanethen-1-yl)-2-(2,6-dichlor-4-trifluormethylthio-phenyl)-hydrazin in 30 ml Ethylenglykolmonoethylether werden 2 Stunden unter Rückfluß erhitzt. Die heiße Lösung wird mit Aktivkohle versetzt, filtriert und mit 60 ml Wasser verdünnt. Der ausgefallene Niederschlag wird abgesaugt und getrocknet. Man erhält 9,8 g (70 % der Theorie) an 5-Amino-4-cyano-1-(2,6-dichlor-4-trifluormethylthiophenyl)-pyrazol vom Schmelzpunkt 185°C bis 187°C.

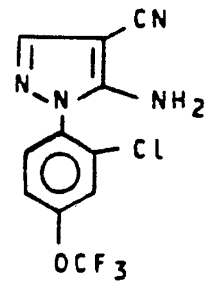

(II-2)

3,08 g (0,025 Mol) Ethoxymethylenmalonsäuredinitril und 5,7 g (0,025 Mol) 2-Chlor-4-trifluormethoxy-phenylhydrazin in 50 ml Ethylenglykolmonoethylether werden 3 Stunden unter Rückfluß erhitzt, nach dem Abkühlen auf Wasser gegossen, der kristaline Niederschlag abgesaugt, mit Petrolether verrührt, gekühlt und abermals abgesaugt.

Man erhält 5,3 g (73,6 % der Theorie) an 5-Amino-4-cyano-1-(2-chlor-4-trifluormethoxyphenyl)-pyrazol vom Schmelzpunkt 115°C.

In entsprechender Weise und gemäß den allgemeinen Herstellungsangaben erhält man die folgenden neuen 5-Aminopyrazole der Formel (II):

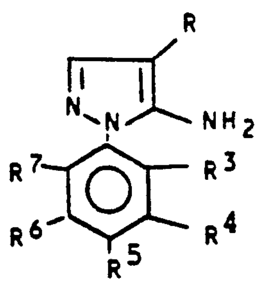

(II)

**Tabelle 3:**

| Bsp. Nr. | R | $R^3$ | $R^4$ | $R^5$ | $R^6$ | $R^7$ | Schmelz-punkt (°C) |
|---|---|---|---|---|---|---|---|
| II-3 | $C_2H_5OCO$- | Cl | H | $-CF_3$ | H | Cl | 134 - 135 |
| II-4 | $C_2H_5OCO$- | Cl | H | $-CF_3$ | H | H | 112 - 114 |
| II-5 | $C_2H_5OCO$- | Cl | H | $-OCF_3$ | H | H | 115 - 118 |
| II-6 | $C_2H_5OCO$- | Cl | H | $-OCF_3$ | H | Cl | 153 - 154 |
| II-7 | CN | Cl | H | $-SCF_3$ | H | H | 159 |
| II-8 | CN | H | Cl | $-SCF_3$ | H | H | 126 - 128 |
| II-9 | CN | H | H | $-SCF_3$ | H | H | 122 |
| II-10 | $C_2H_5OCO$- | H | Cl | $-SCF_3$ | H | H | 120 - 122 |
| II-11 | $C_2H_5OCO$- | Cl | H | $-SCF_3$ | H | H | 129 - 130 |

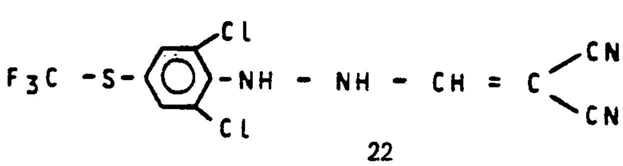

(VI-1)

Zu einer Suspension von 13,9 g (0,05 Mol) (2,6-Dichlor-4-trifluormethylthio)-phenylhydrazin und 2,1 g (0,025 Mol) Natriumacetat in 25 ml Eisessig gibt man unter Rühren 6,1 g (0,05 Mol) Ethoxymethylenmalonsäuredinitril. Nach beendeter Zugabe rührt man eine weitere Stunde bei Raumtemperatur und filtriert den so erhaltenen Feststoff ab, welcher nacheinander mit Wasser, wäßriger Natriumhydrogencarbonatlösung und wieder mit Wasser gewaschen und anschließend getrocknet wird. Man erhält 15,8 g (89 % der Theorie) an 1-(2,2-Dicyanethen-1-yl)-2-(2,6-dichlor-4-trifluormethylthiophenyl)-hydrazin vom Schmelzpunkt 160° C.

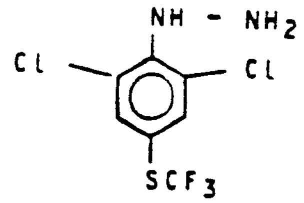

(V-1)

50 g (0,2 Mol) 2,6-Dichlor-4-trifluormethylthio-anilin in 435 ml Eisessig werden bei 55° C bis 60° C zunächst mit 16,6 g (0,24 Mol) Natriummitrit in 150 ml konzentrierter Schwefelsäure und danach bei 5° C bis 10° C mit 180,5 g (0,8 Mol) Zinn-II-chloriddihydrat in 188 ml konzentrierter Salzsäure versetzt. Der so erhaltene Niederschlag wird abgesaugt, in 650 ml eines Gemisches aus Eis und wäßriger Ammoniaklösung verrührt, abgesaugt, getrocknet, zweimal mit je einem Liter Chloroform ausgekocht, filtriert und das Filtrat im Vakuum vom Lösungsmittel befreit. Man erhält 33 g (62,4 % der Theorie) an (2,6-Dichlor-4-trifluormethylthio)-phenylhydrazin vom Schmelzpunkt 58° C.

In entsprechender Weise und gemäß den allgemeinen Herstellungsangaben erhält man die folgenden neuen Zwischenprodukte der Formel (V):

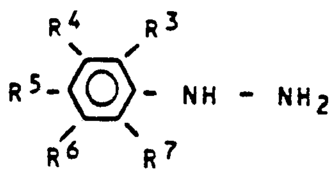

(V)

**Tabelle 4:**

| Bsp. Nr. | $R^3$ | $R^4$ | $R^5$ | $R^6$ | $R^7$ | Schmelzpunkt (° C) |
|---|---|---|---|---|---|---|
| V-2 | Cl | H | $-OCF_3$ | H | H | 35 |
| V-3 | Cl | H | $-OCF_3$ | H | Cl | 61 |
| V-4 | H | Cl | $-OCF_3$ | H | H | 41 - 42 |
| V-5 | H | H | $-OCF_3$ | H | H | Öl, $n^{20}_D = 1,4799$ |
| V-6 | H | H | $-SCF_3$ | H | H | 55 |
| V-7 | H | Cl | $-SCF_3$ | H | H | 72 |
| V-8 | Cl | H | $-SCF_3$ | H | H | 83 |

**Anwendungsbeispiele:**

In den folgenden Anwendungsbeispielen wird die nachstehend aufgeführte Verbindung als Vergleichssubstanz eingesetzt:

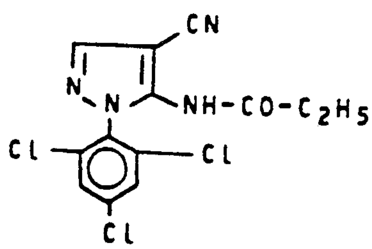

4-Cyano-5-propionylamino-1-(2,4,6-trichlorphenyl)-pyrazol (bekannt aus DE-OS-3 226 513).

**Beispiel A**

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie umbehandelte Kontrolle)
100 % = totale Vernichtung

In diesem Beispiel zeigt z. B. die Verbindung gemäß Herstellungsbeispiel (3) eine deutliche Überlegenheit in der herbiziden Wirksamkeit ebenso wie in der Nutzpflanzenselektivität im Vergleich zum Stand der Technik; dies gilt insbesondere für Weizen.

**Beispiel B**

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

In diesem Beispiel zeigt z. B. die Verbindung gemäß Herstellungsbeispiel (2) eine deutliche Überlegenheit in der herbiziden Wirksamkeit, ebenso wie in der Nutzpflanzenselektivität im Vergleich zum Stand der Technik. Dies gilt ebenfalls insbesondere für Weizen.

**Patentansprüche**

1. 5-Acylamino-1-phenylpyrazole der allgemeinen Formel (I)

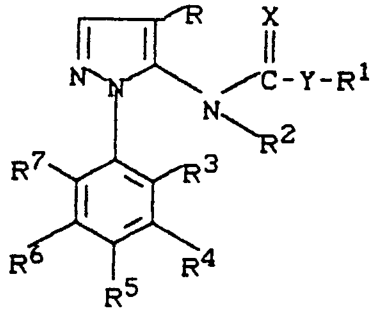

(I)

in welcher
R für Cyano steht,

$R^1$ für jeweils geradkettiges oder verzweigtes Alkyl, Alkenyl, Alkinyl, Alkoxyalkyl oder Alkylthioalkyl, mit jeweils bis zu 6 Kohlenstoffatomen in den einzelnen Alkyl- bzw. Alkenyl- oder Alkinylteilen steht, außerdem für geradkettiges oder verzweigtes Halogenalkyl mit 1 bis 6 Kohlenstoffatomen und 1 bis 9 gleichen oder verschiedenen Halogenatomen steht, ferner für Cycloalkyl mit 3 bis 7 Kohlenstoffatomen oder für gegebenenfalls einfach oder mehrfach, gleich oder verschieden durch Halogen, Cyano, Nitro, jeweils geradkettiges oder verzweigtes Alkyl, Alkoxy, Alkylthio oder Alkoxycarbonyl mit jeweils bis zu 4 Kohlenstoffatomen in den einzelnen Alkylteilen substituiertes Aryl mit 6 bis 10 Kohlenstoffatomen steht,

oder für den Fall, daß Y für die

-N-Gruppe steht,
|
Z

auch für Wasserstoff steht,

X für Sauerstoff oder Schwefel steht,

Y für Sauerstoff, Schwefel oder die

-N-Gruppe steht,
|
Z

wobei Z für Wasserstoff, für jeweils geradkettiges oder verzweigtes Alkyl, Alkoxy, Alkenyl oder Alkinyl mit jeweils bis zu 4 Kohlenstoffatomen oder für gegebenenfalls einfach oder mehrfach, gleich oder verschiedenen durch Halogen, Cyano, Nitro, jeweils geradkettiges oder verzweigtes Alkyl, Alkoxy, Alkylthio oder Alkoxycarbonyl mit jeweils bis zu 4 Kohlenstoffatomen in den einzelnen Alkylteilen substituiertes Aryl mit 6 bis 10 Kohlenstoffatomen steht,

$R^2$ für Wasserstoff oder den Rest

X
||
-C-Y-$R^1$ steht und

$R^3$, $R^4$, $R^5$, $R^6$ und $R^7$ unabhängig voneinander für Wasserstoff, Fluor, Chlor, Brom, Iod, Nitro, jeweils geradkettiges oder verzweigtes Alkyl, Alkoxy, Alkylsulfonyl oder Alkoxycarbonyl mit bis zu 4 Kohlenstoffatomen in den jeweiligen Alkylteilen oder für einen Rest $-(X')_n-R^8$ stehen, wobei

X' für Sauerstoff, Schwefel, Sulfinyl oder Sulfonyl steht,

n für 0 oder 1 steht und $R^8$ für geradkettiges oder verzweigtes Halogenalkyl mit bis zu 4 Kohlenstoffatomen und bis zu 9 gleichen oder verschiedenen Halogenatomen steht, mit der Maßgabe, daß mindestens einer der Reste $R^3$, $R^4$, $R^5$, $R^6$ oder $R^7$ für einen Rest $-(X')_n-R^8$ steht, wobei jedoch nicht gleichzeitig R für Cyano und $R^5$ für Trifluormethyl stehen.

2. Verfahren zur Herstellung von 5-Acylamino-1-phenylpyrazolen der Formel (I)

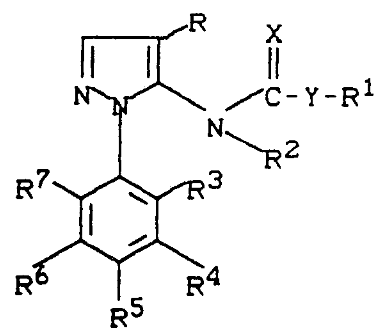

(I)

in welcher

R für Cyano steht,

$R^1$ für jeweils geradkettiges oder verzweigtes Alkyl, Alkenyl, Alkinyl, Alkoxyalkyl oder Alkylthioalkyl, mit jeweils bis zu 6 Kohlenstoffatomen in den einzelnen Alkyl- bzw. Alkenyl- oder Alkinylteilen steht, außerdem für geradkettiges oder verzweigtes Halogenalkyl mit 1 bis 6 Kohlenstoffatomen und 1 bis 9 gleichen oder verschiedenen Halogenatomen steht, ferner für Cycloalkyl mit 3 bis 7 Kohlenstoffatomen oder für gegebenenfalls einfach oder mehrfach, gleich oder verschieden durch Halogen, Cyano, Nitro, jeweils geradkettiges oder verzweigtes Alkyl, Alkoxy, Alkylthio oder Alkoxycarbonyl mit jeweils bis zu 4 Kohlenstoffatomen in den einzelnen Alkylteilen substituiertes Aryl mit 6 bis 10 Kohlenstoffatomen steht,

oder für den Fall, daß Y für die

-N-Gruppe steht,
 |
 Z

auch für Wasserstoff steht,

X für Sauerstoff oder Schwefel steht,

Y für Sauerstoff, Schwefel oder die

-N-Gruppe steht,
 |
 Z

wobei Z für Wasserstoff, für jeweils geradkettiges oder verzweigtes Alkyl, Alkoxy, Alkenyl oder Alkinyl mit jeweils bis zu 4 Kohlenstoffatomen oder für gegebenenfalls einfach oder mehrfach, gleich oder verschiedenen durch Halogen, Cyano, Nitro, jeweils geradkettiges oder verzweigtes Alkyl, Alkoxy, Alkylthio oder Alkoxycarbonyl mit jeweils bis zu 4 Kohlenstoffatomen in den einzelnen Alkylteilen substituiertes Aryl mit 6 bis 10 Kohlenstoffatomen steht,

$R^2$ für Wasserstoff oder den Rest

 X
 ||
-C-Y-$R^1$ steht und

$R^3$, $R^4$, $R^5$, $R^6$ und $R^7$ unabhängig voneinander für Wasserstoff, Fluor, Chlor, Brom, Iod, Nitro, jeweils geradkettiges oder verzweigtes Alkyl, Alkoxy, Alkylsulfonyl oder Alkoxycarbonyl mit bis zu 4 Kohlenstoffatomen in den jeweiligen Alkylteilen oder für einen Rest $-(X')_n-R^8$ stehen, wobei

X' für Sauerstoff, Schwefel, Sulfinyl oder Sulfonyl steht,

n für 0 oder 1 steht und $R^8$ für geradkettiges oder verzweigtes Halogenalkyl mit bis zu 4 Kohlenstoffatomen und bis zu 9 gleichen oder verschiedenen Halogenatomen steht, mit der Maßgabe, daß mindestens einer der Reste $R^3$, $R^4$, $R^5$, $R^6$ oder $R^7$ für einen Rest $-(X')_n-R^8$ steht, wobei jedoch nicht gleichzeitig R für Cyano und $R^5$ für Trifluormethyl stehen,

dadurch gekennzeichnet, daß man

(a) 5-Amino-pyrazole der allgemeinen Formel (II)

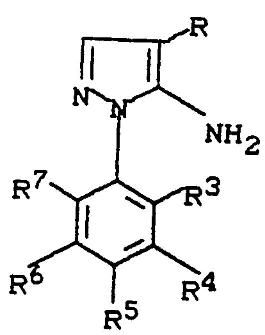

(II)

in welcher

R, $R^3$, $R^4$, $R^5$, $R^6$ und $R^7$ die oben angegebene Bedeutung haben,

(α) mit Acylierungsmitteln der Formel (III)

$R^1$ - Y - C - A
 ||
 X

(III)

in welcher

$R^1$, X und Y die oben angegebene Bedeutung haben und

A für Halogen steht,

gegebenenfalls in Gegenwart eines Verdünnungsmittels und gegebenenfalls in Gegenwart eines Säurebindemittels umsetzt, oder

(β) mit Iso(thio)cyanaten der Formel (IV)

$R^1 - N = C = X$ (IV)

in welcher

$R^1$ und X die oben angegebene Bedeutung haben, ebenfalls gegebenenfalls in Gegenwart eines Verdünnungsmittels und gegebenenfalls in Gegenwart eines basischen Katalysators umsetzt, oder daß man

(b) die nach Verfahren (a - α) erhältlichen Biscarbamate der Formel (Ia)

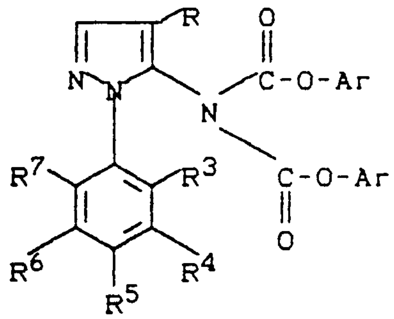

(Ia)

in welcher

Ar für gegebenenfalls ein- bis dreifach gleich oder verschieden durch Halogen, Cyano, Nitro, Methyl, Ethyl, Methoxy, Ethoxy oder Trifluormethyl substituiertes Phenyl steht und

R, $R^3$, $R^4$, $R^5$, $R^6$ und $R^7$ die oben angegebene Bedeutung haben,

mit Alkoholen, Aminen oder Thiolen der Formel (V)

$R^1 - Y - H$ (V)

in welcher

$R^1$ und Y die oben angegebene Bedeutung haben,

gegebenenfalls in Gegenwart eines Verdünnungsmittels und gegebenenfalls in Gegenwart eines basischen Katalysators umsetzt.

3. Herbizide Mittel, gekennzeichnet durch einen Gehalt an mindestens einem 5-Acylamino-1-phenyl-pyrazol der Formel (I) gemäß Anspruch 1 und 2.

4. Verwendung von 5-Acylamino-1-phenylpyrazolen der allgemeinen Formel (I) gemäß Anspruch 1 und 2 zur Bekämpfung von unerwünschtem Pflanzenwachstum.

5. Verfahren zur Herstellung von herbiziden Mitteln, dadurch gekennzeichnet, daß man 5-Acylamino-1-phenylpyrazole der allgemeinen Formel (I) gemäß Anspruch 1 und 2 mit streckmitteln und/oder oberflächenaktiven Mitteln vermischt.

**Revendications**

1. 5-Acylamino-1-phénylpyrazoles de formule génerale (I)

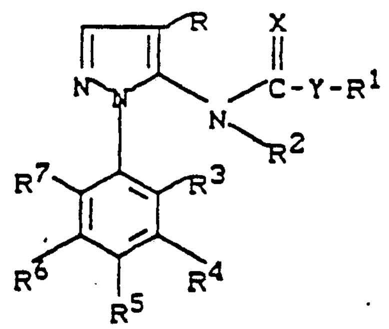

(I)

dans laquelle

R représente un groupe cyano

$R^1$ représente un groupe alkyle, alcényle, alcynyle, alcoxyalkyle ou alkylthioalkyle à chaîne linéaire ou ramifiée, avec jusqu'à 6 atomes de carbone dans chaque partie alkyle, alcényle ou alcynyle, représente également un groupe halogénoalkyle à chaîne linéaire ou ramifiée avec 1 à 6 atomes de carbone et 1 à 9

atomes identiques ou différents d'halogène, représente en outre un groupe cycloalkyle avec 3 à 7 atomes de carbone ou aryle avec 6 à 10 atomes de carbone, éventuellement substitué de manière simple ou multiple, identique ou différente par un halogène, un groupe cyano, nitro, alkyle, alcoxy, alkylthio ou alcoxycarbonyle à chaîne linéaire ou ramifiée avec jusqu'à 4 atomes de carbone dans chaque partie alkyle, ou, dans de cas ou Y représente le groupe

-N-
|
Z

représente également l'hydrogène,
X représente l'oxygène ou le soufre,
Y représente l'oxygène, le soufre ou le groupe

-N-
|
Z

dans lequel Z représente l'hydrogène, un groupe alkyle, alcoxy, alcényle ou alcynyle à chaîne linéaire ou ramifiée avec jusgu'à 4 atomes de carbone ou aryle avec 6 à 10 atomes de carbone, éventuellement substitué de manière simple ou multiple, identique ou différente par un halogène, un groupe cyano, nitro, alkyle, alcoxy, alkylthio ou alcoxycarbonyle à chaîne linéaire ou ramifiée avec jusqu'à 4 atomes de carbone dans chaque partie alkyle,

$R^2$ représente l'hydrogène ou le reste

X
||
$-C-Y-R^1$ et

$R^3$, $R^4$, $R^5$, $R^6$ et $R^7$ représentent, indépendamment les une des autres, l'hydrogène, le fluor, le chlore, le brome, l'iode, un groupe nitro, alkyle, alcoxy, alkylsulfonyle ou alcoxycarbonyle à chaîne linéaire ou ramifiée avec jusqu'à 4 atomes de carbone dans chaque partie alkyle, ou représentent un reste $-(X')_n-R^8$, dans lequel

X, représente l'oxygène, le soufre, un groupe sulfinyle ou sulfonyle,

n représente 0 ou 1 et $R^8$ représente un groupe halogénoalkyle à chaîne linéaire ou ramifiée avec jusqu'à 4 atomes de carbone et jusqu'à 9 atomes identiques ou différents d'halogène, à condition qu'au moins un des restes $R^3$, $R^4$, $R^5$, $R^6$ ou $R^7$ représente un reste $-(X')_n-R^8$, R et $R^5$ ne pouvant toutefois pas représenter simultanément respectivement un groupe cyano et un groupe trifluorométhyle.

2. Procédé de fabrication de 5-acylamino-1-phénylpyrazoles de formule (I)

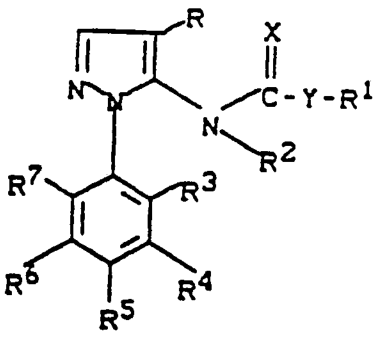

(I)

dans laquelle

R représente un groupe cyano

$R^1$ représente un groupe alkyle, alcényle, alcynyle, alcoxyalkyle ou alkylthioalkyle à chaîne linéaire ou ramifiée, avec jusqu'à 6 atomes de carbone dans chaque partie alkyle, alcényle ou alcynyle, représente également un groupe halogénoalkyle à chaîne linéaire ou ramifiée avec 1 à 6 atomes de carbone et 1 à 9 atomes identiques ou différents d'halogène, représente en outre un groupe cycloalkyle avec 3 à 7 atomes de carbone ou aryle avec 6 à 10 atomes de carbone, éventuellement substitué de manière simple ou multiple, identique ou différente par un halogène, un groupe cyano, nitro, alkyle, alcoxy, alkylthio ou alcoxycarbonyle à chaîne linéaire ou ramifiée avec jusgu'à 4 atomes de carbone dans chaque partie alkyle,

ou, dans le cas où Y représente le groupe

-N-
|
Z

représente également l'hydrogène,

X représente l'oxygène ou le soufre,

Y représente l'oxygène, le soufre ou le groupe

-N-
|
Z

dans lequel Z représente l'hydrogène, un groupe alkyle, alcoxy, alcényle ou alcynyle à chaîne linéaire ou ramifiée avec jusqu'à 4 atomes de carbone ou aryle avec 6 à 10 atomes de carbone, éventuellement substitué de manière simple ou multiple, identique ou différente par un halogène, un groupe cyano, nitro, alkyle, alcoxy, alkylthio ou alcoxycarbonyle à chaîne linéaire ou ramifiée avec jusqu'à 4 atomes de carbone dans chaque partie alkyle,

$R^2$ représente l'hydrogène ou le reste

X
||
$-C-Y-R^1$ et

$R^3$, $R^4$, $R^5$, $R^6$ et $R^7$ représentent, indépendamment uns des autres, l'hydrogène, le fluor, le chlore, le brome, l'iode, un groupe nitro, alkyle, alcoxy, alkylsulfonyle ou alcoxycarbonyle à chaîne linéaire ou ramifiée avec jusqu'à 4 atomes de carbone dans chaque partie alkyle, ou représentent un reste $-(X')_n-R^8$, dans lequel

X' représente l'oxygène, le soufre, un groupe sulfinyle ou sulfonyle,

n représente 0 ou 1 et $R^6$ représente un groupe halogénoalkyle à chaîne linéaire ou ramifiée avec jusqu'à 4 atomes de carbone et jusqu'à 9 atomes identiques ou différents d'halogène, à condition qu'au moins un des restes $R^3$, $R^4$, $R^5$, $R^6$ ou $R^7$ représente un reste $-(X')_n-R^8$, R et $R^5$ ne pouvant toutefois pas représenter simultanément respectivement un groupe cyano et un groupe trifluorométhyle,

caractérisé en ce que l'on fait réagir

(a) des 5-amino-pyrazoles de formule générale (II)

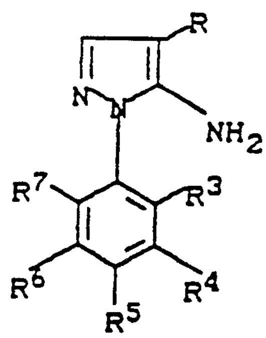

(II)

dans laquelle

R, $R^3$, $R^4$, $R^5$, $R^6$ et $R^7$ ont la signification décrite ci-dessus,

(α) avec des agents d'acylation de formule (III)

$R^1$ - Y - C - A
||
X

(III)

dans laquelle

$R^1$, X et Y ont la signification décrite ci-dessus et

A représente un halogène

éventuellement en présence d'un agent de dilution et éventuellement en présence d'un agent liant de l'acide, ou

(β) avec des iso(thio)cyanates de formule (IV)

$R^1 - N = C = X$ (IV)

dans laquelle

$R^1$ et X ont la signification décrite ci-dessus, aussi éventuellement en présence d'un agent de dilution et éventuellement en présence d'un catalyseur basique, ou en ce que l'on fait réagir

(b) les biscarbamates de formule (Ia)

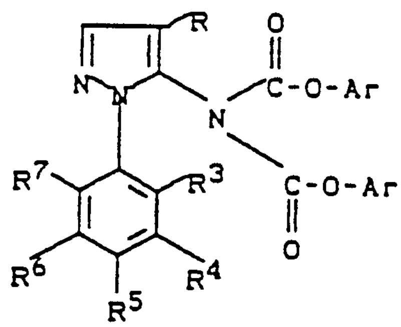

(Ia)

pouvant être obtenus suivant le procédé (a - α), dans laquelle

Ar représente un groupe phényle éventuellement substitué de manière simple ou triple, identique ou différente par un halogène, un groupe cyano, nitro, méthyle, éthyle, méthoxy, éthoxy ou trifluorométhyle et

R, $R^3$, $R^4$, $R^5$, $R^6$ et $R^7$ ont la signification décrite ci-dessus,

avec des alcools, des amines ou des thiols de formule (V)

$$R^1 - Y - H \qquad (V)$$

dans laquelle

$R^1$ et Y ont la signification décrite ci-dessus, eventuellement en présence d'un agent de dilution et éventuellement en présence d'un catalyseur basique.

3. Agents herbicides caractérisés par une teneur d'au moins un 5-acylamino-1-phényl-pyrazole de formule (I) suivant les revendications 1 et 2.

4. Utilisation de 5-acylamino-1-phénylpyrazoles de formule générale (I) suivant les revendications 1 et 2 pour lutter contre la croissance indésirable de plantes.

5. Procédé de fabrication d'agents herbicides caractérisé en ce que l'on mélange des 5-acylamino-1-phénylpyrazoles de formule générale (I) suivant les revendications 1 et 2 avec des diluants et/ou des agents tensio-actifs.

## Claims

1. 5-Acylamino-1-phenylpyrazoles of the general formula (I)

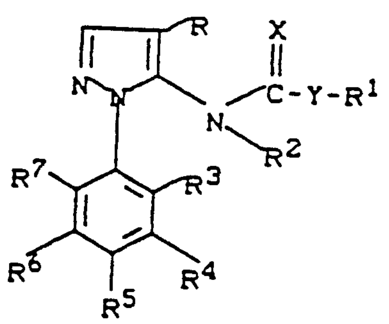

(I)

in which

R represents cyano

$R^1$ represents alkyl, alkenyl, alkinyl, alkoxyalkyl or alkylthioalkyl, each of which is straight-chain or branched and each of which has up to 6 carbon atoms in the individual alkyl or alkenyl or alkinyl parts, and furthermore represents straight-chain or branched halogenoalkyl having 1 to 6 carbon atoms and 1 to 9 identical or different halogen atoms, and furthermore represents cycloalkyl having 3 to 7 carbon atoms, or aryl which has 6 to 10 carbon atoms and is optionally monosubstituted or polysubstituted by identical or different substituents, suitable substituents being: halogen, cyano, nitro, or alkyl, alkoxy, alkylthio or alkoxycarbonyl, each of which is straight-chain or branched and each of which has up to 4 carbon atoms in the individual alkyl parts,

or in the case in which Y represents the

-N- group,
|
Z

also represents hydrogen,
X represents oxygen or sulphur,
Y represents oxygen, sulphur or the

-N- group,
|
Z

wherein
Z represents hydrogen or alkyl, alkoxy, alkenyl or alkinyl, each of which is straight-chain or branched and each of which has up to 4 carbon atoms, or represents aryl which has 6 to 10 carbon atoms and is optionally monosubstituted or poly-substituted by identical or different substituents, suitable substituents being: halogen, cyano, nitro, or alkyl, alkoxy, alklthio or alkoxycarbonyl, each of which is straight-chain or branched and each of which has up to 4 carbon atoms in the individual alkyl parts X
$R^2$ represents hydrogen or the radical

X
||
$-C-Y-R^1$ and

$R^3$, $R^4$, $R^5$, $R^6$ and $R^7$ independently of one another represent hydrogen, fluorine, chlorine, bromine, iodine or nitro, or alkyl, alkoxy, alkylsulphonyl or alkoxycarbonyl, each of which is straight-chain or branched and has up to 4 carbon atoms in the particular alkyl parts, or represents a radical $-(X')_n-R^8$,
wherein
X' represents oxygen, sulphur, sulphinyl or sulphonyl,
n represents 0 or 1 and
$R^8$ represents straight-chain or branched halogenoalkyl having up to 4 carbon atoms and up to 9 identical or different halogen atoms, with the proviso that at least one of the radicals $R^3$, $R^4$, $R^5$, $R^6$ or $R^7$ represents a radical $-(X')_n-R^8$, but R does not represent cyano when $R^5$ represents trifluoromethyl.

2. Process for the preparation of 5-acylamino-1-phenylpyrazoles of the formula (I)

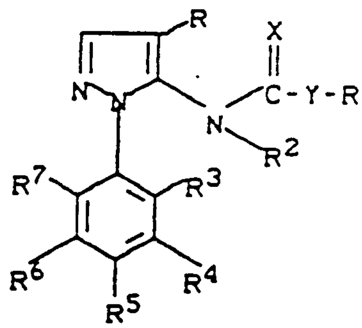

(I)

in which
R represents cyano
$R^1$ represents alkyl, alkenyl, alkinyl, alkoxyalkyl or alkylthioalkyl, each of which is straight-chain or branched and each of which has up to 6 carbon atoms in the individual alkyl or alkenyl or alkinyl parts, and furthermore represents straight-chain or branched halogenoalkyl having 1 to 6 carbon atoms and 1 to 9 identical or different halogen atoms, and furthermore represents cycloalkyl having 3 to 7 carbon atoms, or aryl which has 6 to 10 carbon atoms and is optionally mono-substituted or polysubstituted by identical or different substituents, suitable substituents being: halogen, cyano, nitro, or alkyl, alkoxy, alkylthio or alkoxycarbonyl, each of which is straight-chain or branched and each of which has up to 4 carbon atoms in the individual alkyl parts,
or in the case in which Y represents the

-N- group,
|
Z

also represents hydrogen,
X represents oxygen or sulphur,
Y represents oxygen, sulphur or the

-N- group,
|
Z

wherein
Z represents hydrogen or alkyl, alkoxy, alkenyl or alkinyl, each of which is straight-chain or branched and each of which has up to 4 carbon atoms, or represents aryl which has 6 to 10 carbon atoms and is optionally monosubstituted or poly-substituted by identical or different substituents, suitable substituents being: halogen, cyano, nitro, or alkyl, alkoxy, alklthio or alkoxycarbonyl, each of which is straight-chain or branched and each of which has up to 4 carbon atoms in the individual alkyl parts X
$R^2$ represents hydrogen or the radical

X
||
$-C-Y-R^1$ and

$R^3$, $R^4$, $R^5$, $R^6$ and $R^7$ independently of one another represent hydrogen, fluorine, chlorine, bromine, iodine or nitro, or alkyl, alkoxy, alkylsulphonyl or alkoxycarbonyl, each of which is straight-chain or branched and has up to 4 carbon atoms in the particular alkyl parts, or represents a radical $-(X'')_n-R^8$,
wherein
X' represents oxygen, sulphur, sulphinyl or sulphonyl,
n represents 0 or 1 and
$R^8$ represents straight-chain or branched halogenoalkyl having up to 4 carbon atoms and up to 9 identical or different halogen atoms, with the proviso that at least one of the radicals $R^3$, $R^4$, $R^5$, $R^6$ or $R^7$ represents a radical $-(X')_n-R^8$, but R does not represent cyano when $R^5$ represents trifluoromethyl,
characterized in that
(a) 5-amino-pyrazoles of the general formula (II)

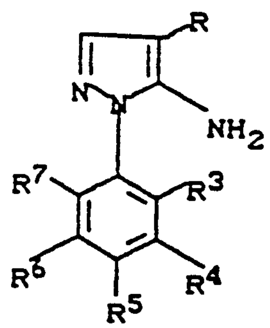
(II)

in which
R, $R^3$, $R^4$, $R^5$, $R^6$ and $R^7$ have the meaning given above,
(α) are reacted with acylating agents of the formula (III)

$$R^1-Y-\underset{\underset{X}{||}}{C}-A \qquad (III)$$

in which
$R^1$, X and Y have the meaning given above and
A represents halogen,
if appropriate in the presence of a diluent and, if appropriate, in the presence of an acid-binding agent, or (β) are reacted with iso(thio)cyanates of the formula (IV)

$$R^1-N=C=X \qquad (IV)$$

in which

$R^1$ and X have the meaning given above, if appropriate also in the presence of a diluent and, if appropriate, in the presence of a basic catalyst, or in that (b) the biscarbamates obtainable by process (a - α), of the formula (Ia)

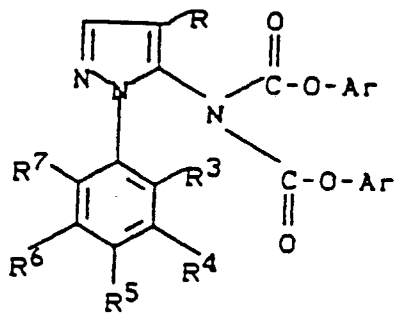

(Ia)

in which

Ar represents phenyl which is optionally monosubstituted to trisubstituted by indentical or different substituents from amongst halogen, cyano, nitro, methyl, ethyl, methoxy, ethoxy or trifluoromethyl and

R, $R^3$, $R^4$, $R^5$, $R^6$ and $R^7$ have the meaning given above,

are reacted with alcohols, amines or thiols of the formula (V)

$$R^1 - Y - H \qquad (V)$$

in which

$R^1$ and Y have the meaning given above,

if appropriate in the presence of a diluent and, if appropriate, in the presence of a basic catalyst.

3. Herbicidal agents, characterized in that they contain at least one 5-acylamino-1-phenyl-pyrazole of the formula (I) according to Claims 1 and 2.

4. Use of 5-acylamino-1-phenylpyrazoles of the general formula (I) according to Claims 1 and 2 for combating undesired plant growth.

5. Process for the preparation of herbicidal agents, characterized in that 5-acylamino-1-phenylpyrazoles of the general formula (I) according to Claims 1 and 2 are mixed with extenders and/or surface-active agents.